(19) Europäisches Patentamt
European Patent Office
Office européen des brevets

(11) **EP 3 640 947 A1**

(12) **EUROPÄISCHE PATENTANMELDUNG**

(43) Veröffentlichungstag:
**22.04.2020 Patentblatt 2020/17**

(51) Int Cl.:
***G16C 20/10*** *(2019.01)*

(21) Anmeldenummer: **18201340.9**

(22) Anmeldetag: **18.10.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **MONTE-CARLO-METHODE ZUR AUTOMATISIERTEN UND HOCH-EFFIZIENTEN BERECHNUNG VON KINETISCHEN DATEN CHEMISCHER REAKTIONEN**

(57) Die vorliegende Erfindung betrifft ein computerimplementiertes Verfahren zur Berechnung von Übergangszustanden einer chemischen Reaktion, sowie ein System zur Datenverarbeitung umfassend Mittel zur Ausführung des Verfahrens, ein Computerprogramm umfassend Befehle die einen Computer veranlassen das Verfahren durchzuführen und die Verwendung des Computerprogramms

**EP 3 640 947 A1**

**EP 3 640 947 A1**

## Beschreibung

Die vorliegende Erfindung betrifft ein computerimplementiertes Verfahren zur Berechnung von Übeigangszuständen einer chemischen Reaktion, sowie ein System zur Datenverarbeitung umfassend Mittel zur Ausführung des Verfahrens, ein Computerprogramm umfassend Befehle die einen Computer veranlassen das Verfahren durchzuführen und die Verwendung des Computerprogramms

Während einer chemischen Reaktion verändern die beteiligten Atome ihre Geometrie und Bindungen werden gebrochen und neue Bindungen gebildet. Dabei verandert sich auch die Energie der beteiligten Atome und erreicht während des Verlaufs der chemischen Reaktion einen Zustand maximaler Energie, den sogenannten Übergangszustand Der Übergangszustand stellt einen Potentialwall oder eine Aktivierungsbarriere dar, der die Edukte von den Produkten der chemischen Reaktion trennt. Ist die Aktivierungsbarriere überwunden, so wird das Produkt gebildet Die Energie während einer chemischen Reaktion kann mit Hilfe einer sogenannten Potentialhyperfläche dargestellt werden, welche die potentielle Energie der an der Reaktion beteiligten Atome in Abhängigkeit von ihrer Geometrie abbildet Dabei bildet die Potentialhyperfläche auch Zustande ab, bei denen keine Produkte gebildet werden Der direkte Reaktionspfad, bei dem Produkte unter Überwindung des Ubergangszustandes gebildet werden, kann als Kurve gezeigt werden, bei der die Abstände der einzelnen Atome der Moleküle gegen die Energie aufgetragen werden Mit Hilfe von quantenchemischen Methoden und mathematischen Näherungsverfahren kann die Energie der Geometrie eines Moleküls oder eines Systems von mehreren Molekulen berechnet werden, wobei der dabei zu betrachtende Funktionsraum die Freiheitsgrade des Moleküls umfasst. Diese Verfahren ermöglichen die Ermittlung der Geometrie am Ubergangszustand und erlauben Vorhersagen über die Reaktionskinetik einer chemischen Reaktion.

Eine bekannte Methode mit der die Geometrie am Übergangszustand ermittelt werden kann, sind Quasi-Newton-Raphson-Verfahren, auch Pseudo-Newton-Raphson Verfahren genannt. Die Berechnung von Übergangszuständen mit Hilfe von Quasi-Newton-Raphson-Verfahren weist jedoch einige Nachteile auf Zunachst fuhren Quasi-Newton-Raphson Algorithmen nicht bei jeder beliebigen Ausgangsgeometrie eines Molekül zu einem Übergangszustand sondern nur bei Molekülgeometrien, die bereits sehr nah an die Geometrie des Übergangszustandes angenähert sind Die Annaherung ist aufwendig, weil die Annaherung einer Molekulgeometrie an die Geometrie des Ubergangszustandes üblicherweise händisch erfolgt, d.h es werden Bindungslangen per Hand am Computer eingestellt Es sind auch Methoden bekannt, bei denen zunächst mit anderen Methoden Potentialhyperflachen berechnet werden aus denen mogliche Sattelpunkte identifiziert werden Auch bei diesen Methoden muss also eist eine Geometrie ermittelt werden, die bereits sehr nah an die Geometrie des Ubergangszustandes angenahert ist Des Weiteren sind Quasi-Newton-Raphson Verfahren anfallig für Fehler wenn sie auf derartige, händisch ausgewahlte Molekulgeometrien angewendet werden Pseudo-Newton-Raphson basierte Verfahren führen oft dazu, dass an Stelle der benotigten Sattelpunkte erster Ordnung lokale Minima erhalten werden Das liegt daran, dass in der Theorie der Newton-Raphson basierten Methoden der Übergangszustand selbst in einer Potenzieihe an der Stelle Gradient=0 entwickelt wird Ist die händisch generierte Geometrie zu weit vom Ubergangszustand selbst entfernt, ist die Voraussetzung Gradient≈0 nicht ausieichend erfüllt um eine Konvergenz zu erreichen Außerdem wird in Quasi-Newton-Raphson Verfahren die Hessematnx nur im ersten Schutt berechnet. In den folgenden Schritten wird diese durch Update-Algorithmen geschätzt, was eine Fehlerquelle darstellt. Je weiter die Anfangsstruktur vom Übergangszustand entfernt ist, umso mehr Iterationen bzw Updates werden benötigt, desto großer ist der Fehler oder Abstand zur richtigen Berechnung der Hessematrix, und desto weiter ist der Abstand zu einer Konvergenz.

Lin et al ("A flexible transition state searching method for atmospheric reaction systems," Chemical Physics 450-451, 2015, S 21-31) offenbaren eine Methode zur Untersuchung atmosphärischer chemischer Reaktionen in der Gasphase. Die Methode umfasst m emem ersten Schritt ein auf Monte-Carlo-Methoden basiertes Screening von Potentialhyperflächen mit Kraftfeld-Methoden, wobei genaherte Sattelpunkt-artige Regionen identifiziert werden Die verwendete Observable der entsprechenden Monte Carlo Methode ist dabei der Wert einer Energiefunktion Darauf aufbauend wird dann versucht mittels einer Quasi Newton-Raphson Methode-chemische Übeigangszustände zu lokalisieren Mit der hier offenbarten Monte-Carlo-Methode muss zunächst die gesamte Potentialhyperflache simuliert werden, bevor uberhaupt genaherte Sattelpunkt-artige Regionen identifiziert werden können Folglich konnen mit dieser Monte-Carlo-Methode nicht gezielt Sattelpunkt-artige Regionen optimiert werden. Aus diesem Grund ist keine effiziente Nutzung der verwendeten Rechenressourcen zur Lokalisierung der benotigten Sattelpunkte möglich. Der Einsatz der hier offenbarten Methode beschränkt sich mit der derzeitigen Computertechnik auf kleine Molekule mit bis zu 30 Atomen Zudem werden mathematisch stark vereinfachte Methoden verwendet

E. Martínez-Núñez et al "An automated transition state search using classical trajectories initialized at multiple minima", Phys Chem Chem Phys, 14. Juni 2015, 17(22).14912-21, "tsscds2018 A code for automated discovery of chemical reaction mechanisms and solving the kinetics", J. Comp.Chem., 24 September 2018, 39(23)1922-1930) offenbaren eine Methode zur Untersuchung chemische Reaktionspfade Dabei werden Potentialhyperflächen chemischer Molekule mit mathematisch vereinfachten Methoden berechnet. Anschließend wird versucht die chemisch relevanten Übergangszustande mit gewöhnlichen Pseudo-Newton-Raphson-Methoden zu berechnen. Auch hier ist nachteilig, dass zunachst die gesamte Potentialhyperflache berechnet werden muss, was zeitaufwandig ist und Rechnerleistung braucht Zudem kann auch diese Methode nur auf kleinere Molekule angewendet weiden

Jacobson et al ("Automated Transition State Search and Its Application to Diverse Types of Organic Reactions", J. Chem Theory Comput. 13, 11, 5780-5797) offenbaren eine Methode zur automatisierten Berechnung von chemischen Übergangszustanden Dabei weiden chemische Übergangszustande aus chemischen Gleichgewichtszustanden berechnet. Dieses Vorgehen benotigt eine separate Berechnung von chemischen Gleichgewichtszustanden, bevor eine Interpolation der erhaltenen Geometrien durchgeführt werden kann, mit welchen der Übergangszustand angenähert wird In einem weiteren Schritt wird dann basierend auf dieser Annaherung versucht eine Übergangszustandsgeometrie zu berechnen Das Erstellen der benotigten Glerchgewichtszustande erfordert zusätzlichen Arbeitsaufwand durch den Fachmann. Die in der offenbarten Methode verwendete Interpolation lasst sich nicht mit Erfolg auf beliebige Molekulgeometrien anwenden.

Hu et al ("A gradient-directed Monte Carlo method for global optimization in a discrete space. Application to protein sequence design and folding" J Chem Phys 2009 Oct 21, 131(15)·154117) offenbaren eine Methode zur Berechnung von Proteinstrukturen offenbart. Bei dieser Methode werden Faltungen von Proteinstrukturen mittels Monte-Carlo-Methoden berechnet Zur Beschleunigung der Konvergenz der Monte-Carlo-Prozeduren werden Gradienten berechnet, welche in die Richtung und Größe von Auslenkungen der Atompositionen eingehen Die Publikation lasst keine mögliche Verwendung dieser Methode zur gezielten Berechnung von Sattelpunkten erkennen.

Nachteilig an den bekannten Verfahren zur Berechnung von Übergangständen ist die Kombination unterschiedlicher quantenchemischer Verfahren zur Berechnung einer Potentialhyperflache oder einer Ausgangsgeometrie einerseits und zur Berechnung des Sattelpunktes andererseits Die Potentralhyperflache, die mit einem quantenchemischen Verfahren berechnet wird, ist nicht unbedingt geeignet ist den Sattelpunkt mit einem anderen quantenchemischen Verfahren anderer Qualitat zu berechnen, da unterschiedliche quantenchemische Methoden Parameter bei der Naherung der Potentialhyperfläche unterschiedliche Genauigkeiten ergeben. Daher sind die mit diesen Verfahren erhaltenen Sattelpunkte häufig ungenau oder sogar weit vom tatsächlichen Sattelpunkt entfernt. Des Weiteren ist die händische Annaherung einer Molekülgeometrie an die Geometi ie des Übergangszustandes zeit- und personalaufwendig.

Es besteht somit ein Bedarf für ein Verfahren für die Berechnung von Übergangszustanden, bei dem eine Molekulgeometrie für eine Ubergangszustands einer chemischen Reaktion mit einem einfachen Verfahren, insbesondere einem computerimplementierten Verfahren, genähert werden kann, bevor mit einer Berechnung des Ubergangszustands mit Hilfe eines quantenchemischen Verfahrens, insbesondere eines Pseudo-Newton-Raphson-Verfahrens, begonnen werden kann. Insbesondere besteht ein Bedarf für ein Verfahren mit dem Übergangszustande ermittelt werden konnen, ohne zuvor eine Potentialhyperflache berechnen zu müssen

Aufgabe der vorliegenden Erfindung war ein computerimplementiertes Verfahren zur Verfügung zu stellen, bei dem eine Geometrie eines Ubergangszustandes mit einem leicht anwendbaren, insbesondere einem computerimplementierten, Verfahren angenähert werden kann, bevor in einem folgenden Schritt der Übergangszustand mit Hilfe von quantenchemischen Verfahren, insbesondere von Pseudo-Newton-Raphson-Algorithmen, berechnet wird. Insbesondere soll eine Molekulgeometrie für einen Übergangszustand einer chemischen Reaktion ermittelt werden können, ohne zuvor die Potentialhyperflache der chemischen Reaktion zu berechnen

Diese Aufgabe wurde gelöst durch ein computer implementiertes Verfahren zur Berechnung von Ubergangszustanden einer chemischen Reaktion umfassend die Schritte

### A Erzeugen einer Startgeometrie

A1 Bereitstellung der dreidimensionalen Darstellung mindestens eines Moleküls im energetischen Grundzustand,

A2 Auswahl von mindestens einer Bindung des mindestens einen Moleküls und Auswahl einer Lange der Bindung, wobei die ausgewählte Lange nicht der Lange der Bindung im energetischen Grundzustand des Moleküls entspricht, so dass eine Startgeometrie für die chemische Reaktion erhalten wird,

A3 Dreidimensionale Darstellung der Startgeometrie in kartesischen , und/oder internen Koordinaten,

### B Ermittlung einer optimierten Startgeometrie

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewahlten Funktionsraums mittels einer quantenchemischen Methode und mit der Randbedingung, dass die in Schritt A2 ausgewählte Lange der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Molekuls in der optimierten Startgeometrie,

> B4.1 wenn die Gradient-Norm B3 $\nabla$ $0 \leq \nabla \leq 0{,}07$ $E_h$ $a_0^{-1}$ betragt, dann Einordnung der optimierten Startgeometrie als eine Vorstufe für den Übergangszustand der chemischen Reaktion und Fortführung des Verfahrens mit Schritt D1, oder
> B4 2 wenn die Gradient-Norm B3 $\nabla > 0{,}07$ $E_h$ $a_0^{-1}$ betragt, dann Ermittlung der Vorstufe für den Übergangszustand der chemischen Reaktion ausgehend von der optimierten Startgeometrie durch ein Verfahren umfassend die folgenden Schritte·

**C Ermittlung der Vorstufe für den Übergangszustand der chemischen Reaktion**

> C1 Variation der optimierten Startgeometrie unter Anwendung eines Monte-Carlo-Algorithmus wobei

>> C1 1 wenigstens ein Atom aus dem in Schritt B1 ausgewählten Funktionsraum zufällig ausgewählt wird,
>> C1.2 ein Vektor für eine Auslenkung des in Schritt C1 1 gewahlten Atoms zufällig ausgewählt wird,
>> C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

> C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1 3 ermittelt wurde,
> C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Ubergangszustand und x=Kernkoordinaten des Molekuls in der Vorstufe für den Übergangszustand,
> C4 1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder
> C4 2 wenn die Gradient-Norm C3 $\nabla > 0{,}07$ $E_h$ $a_0^{-1}$ betragt, dann Wiederholung der Schritte C1 bis C3 bis eine Gradient-Norm C3 $0 \leq \nabla \leq 0{,}07$ $E_h$ $a_0^{-1}$ erhalten wird, wobei

>> (a) für den Fall, dass die Schritte C1 bis C3 ein Mal durchgeführt wurden, in Schritt C1 die Geometrie der Vorstufe für den Ubergangszustand variiert wird, wenn der Wert ihrer Gradient-Norm C3 niedriger ist als der Wert der Gradient-Norm B3 der optimierten Startgeometrie, oder

>> (b) für den Fall dass die Schritte C1 bis C3 mehr als ein Mal durchgeführt wurden, in Schritt C1 die Geometrie der optimierten Startgeometrie oder derjenigen Vorstufe für den Ubergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

> D1 Relaxation der Vorstufe aus Schritt C4 1 oder der Vorstufe aus Schritt B4 1 mittels der quantenchemischen Methode und eines Pseudo-Newton-Raphson-Algorithmus, so dass der Ubergangszustand erhalten wird,
> D2 gegebenenfalls Ermittlung eines Gleichgewichtszustands durch Auslenkung des Ubergangszustands, so dass ein ausgelenkter Ubergangszustand erhalten wird und Relaxation des ausgelenkten Übergangszustands mittels der quantenchemischen Methode, so dass ein Gleichgewichtszustand erhalten wird

Es wurde uberraschend gefunden, dass die Aufgabe gelost werden kann, indem durch Einsatz einer geeigneten Monte-Carlo-Methode verschiedene Molekulgeometrien variiert weiden und mit Hilfe eines Gütekriteriums eine Geometrie für einen Übergangszustand angenahert wird Des Weiteren wurde überraschend gefunden, dass die Aufgabe gelöst werden kann, indem bei dem Monte-Carloverfahren als Observable der Gradient-Norm der Kurve des direkten Reaktionspfades verwendet wird. Dadurch lässt sich der zu behandelnde Funktionsraum, der die Molekulgeometrie am Übergangszustand der chemischen Reaktion abbilden soll, deiart reduzieren, dass die Berechnung von Sattelpunkten ermoglicht wird, ohne zuvor eine Potentialhyperfläche zu berechnen oder manuell Molekülgeometrien zu suchen Außerdem wurde überraschend gefunden, dass mit dem erfindungsgemäßen Verfahren die Berechnung von Sattelpunkten in hochdimensionalen Funktionsraumen möglich wird. Ein weiterer Vorteil des erfindungsgemäßen Verfahrens ist, dass die quantenchemischen Berechnungen bei der Durchführung des Verfahrens mit der gleichen Methode durchgeführt werden. Mit der derzeitigen Computertechnik können mit dem erfindungsgemäßen Verfahren deutlich kürzer und schneller auch Ubergangszustände für Moleküle mit bis zu 100 Atomen berechnet werden.

In **Schritt A** des erfindungsgemäßen Verfahrens wird zunächst eine Startgeometrie erzeugt, indem in Schritt A1 die dreidimensionale Darstellung mindestens eines Moleküls im energetischen Grundzustand bereitgestellt wird Dann wird in Schritt A2 eine Bindung des Molekuls ausgewahlt und eine Länge der Bindung, die von der Länge der Bindung im energetischen Grundzustand abweicht, so dass eine Startgeometrie erhalten wird. Bevorzugt weist das mindestens eine Molekul aus Schritt A1 eine Große von hochstens 100 Atomen auf, weiter bevorzugt von hochstens 80 Atomen, bevorzugter von hochstens 60 Atomen und/oder betragt die Länge der in Schritt **A2** ausgewahlten Bindung höchstens 230 pm, bevorzugt hochstens 200 pm, weiter bevorzugt hochstens 180 pm, bevorzugter 150 pm

In einer weiteren bevorzugten Ausführungsform des erfindungsgemäßen Verfahrens weiden in Schi itt **A1** mindestens zwei Molekule I und II bereitgestellt und in Schritt **A2** können alternativ oder zusätzlich zu der mindestens einen Bindung auch mindestens ein Abstand zwischen mindestens einem Atom aus Molekul I und mindestens emem Atom aus Molekül II sowie die Länge des mindestens einen Abstands ausgewahlt werden, wobei die Lange des Abstands insbesondere hochstens 230 pm betragt, bevorzugt hochstens 200 pm, weiter bevorzugt hochstens 180 pm, bevorzugter 150 pm. Wenn das Verfahren mit mindestens zwei Molekulen I und II durchgeführt wird und die Länge des Abstands zwischen Atomen der verschiedenen Molekule ausgewählt wird, so kann bevorzugt der Ubergangszustand eine Synthesereaktion mit dem Verfahren berechnet werden Bevorzugt weist Molekul I eine Große von $\leq 100$ Atomen auf und Molekül II eine Größe von $\leq 100$ Atomen auf, bevorzugter weist Molekül I eine Größe von $\leq 80$ Atomen auf und Molekul II eine Größe von $\leq 80$ Atomen auf, noch bevorzugter weist Molekul I eine Größe von $\leq 60$ Atomen auf und Molekül II eine Große von $\leq 60$ Atomen auf Bevorzugt beträgt die Summe der Atome aus Molekul I und aus Molekül II $\leq 100$ Atome

Bevorzugt ist Molekul I ein Katalysator für die chemische Reaktion, insbesondere für eine Polymersynthese, und Molekul II ein Edukt der chemischen Reaktion. Dabei handelt es sich bei der Polymersynthese in dieser Ausführungsform bevorzugt um Polyurethansynthesen Die chemische Reaktion in dieser Ausführungsform sind bevorzugt auch Synthesen von Monomeren für Polymerisationsreaktionen, industriell benötigte Grundchemikalien, Additiven, Tensiden und pharmakologischen Wirkstoffen Insbesondere ist die chemische Reaktion eine Synthese für mit Katalysatoren erhaltene Grundchemikalien oder Edukte für chemische Synthesen Unter Additiven werden allgemein Additive für Kunststoffe wie Weichmacher, Antioxidantien, Stellmittel verstanden, sowie Zusätze für Kraftstoffe, bei deren Synthese jeweils Katalysatoren verwendet werden.

In Schritt A3 wird die ausgewählte Startgeometrie in kartesischen und/oder internen Koordinaten dargestellt Interne Koordinaten beschreiben die raumliche Anordnung der Atome relativ zueinander unter Benutzung von Bindungslangen, Bindungswinkel und Torsionswinkel.

In **Schritt B** des erfindungsgemäßen Verfahrens wird eine optimierte Startgeometrie ermittelt Dazu wird zunachst in Schritt B1 ein Funktionsraum festgelegt. Der Funktionsraum umfasst die Raumkoordinaten von ausgewählten Atomen, wobei die Raumkoordinaten im Vektorraum aller im Molekul beinhalteten Atomkoordinaten einen Unterraum aufspannen Die ausgewählten Atome für den Funktionsraum sind ein Satz an Atomen, die an einer Bindungsdissoziation beteiligt sind oder bevorzugt an einer Synthesereaktion Des Weiteren umfasst der Funktionsraum die mindestens eine Bindung aus Schritt A2 Die Bei der bevorzugten Ausführungsform mit mindestens zwei Molekulen I und II umfasst der Funktionsraum den Abstand zwischen mindestens einem Atom aus Molekul I und mindestens einem Atom aus Molekül II

Im folgenden Schritt B2 wird die Startgeometrie einer Geometrieoptimierung mittels einer quantenchemischen Methode unterzogen mit der Randbedingung, dass die in Schritt A2 ausgewahlte Länge der mindestens einen Bindung konstant gehalten wird, so dass eine optimierte Startgeometrie erhalten wird. Dabei umfasst die Geometrieoptimierung alle Atome des als Startgeometrie ausgewahlten Molekuls Bei der bevorzugten Ausführungsform mit mindestens zwei Molekulen I und II wird bei der Geometrieoptimierung der Abstand zwischen mindestens einem Atom aus Molekul I und mindestens einem Atom aus Molekul II konstant gehalten, so dass eine optimierte Startgeometrie erhalten wird. Bei der Geometrieoptimierung mit Randbedingung wird die Gesamtenergie des Moleküls als Funktion der Kernkoordinaten der im Molekül enthaltenen Atome mit einem quantenchemischen Verfahren minimiert. Um ein lokales Energiemmimum zu erhalten wird die Energie nach Gradienten minimiert (zb. Steepest Descent), wobei die Energie soweit minimiert wird, wie die Randbedingung dies zulässt.

In Schritt B3 wird die Gradient-Norm B3 für die zuvor erhaltene optimierte Startgeometrie ermittelt, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie In Schritt B3 wird die gleiche quantenchemische Methode verwendet wie in Schritt B2 Die euklidische Norm dieses Vektors ist die Gradient-Norm. Mit anderen Worten wird der Gradient-Vektor der Energie berechnet. Der Gradient-Vektor spannt denselben vektoriellen Raum auf wie das Molekül. Jede Komponente des Vektors besteht aus der partiellen Ableitung der Energie in der $x_1$-Koordinate $\left(\frac{\partial}{\partial x_t} E(x)\right)$ und spannt sich in die Richtung des Einheitsvektors der $x_1$-Koordinate. Danach wird die (euklidische) Norm dieses Vektors ermittelt

Die weitere Fortführung des Verfahrens von der Große der erhaltenen Gradient-Norm B3 ab Sofern die Gradient-Norm B3 $\nabla$ $0 \leq \nabla \leq 0{,}07$ $E_h$ $a_0^{-1}$ beträgt, dann kann die optimierten Startgeometrie als eine Vorstufe für den Übergangszustand der chemischen Reaktion eingeordnet werden und Schritt C des Verfahrens ausgelassen werden und das Verfahren mit Schritt D1 fortgeführt werden. Betragt die Gradient-Norm B3 $\nabla > 0{,}07$ $E_h$ $a_0^{-1}$, dann wird Schritt C des Verfahrens durchgeführt und zunächst eine Vorstufe für den Ubergangszustand der chemischen Reaktion ermittelt und zwar ausgehend von der optimierten Startgeometrie Dabei steht $E_h$ für die Hartree Energie, wobei 1 $E_h$ = 4 3597 · $10^{-18}$ J, und $a_0$ steht für den Bohr-Radius, wobei 1 $a_0$ = 5.29 $10^{-11}$ m

In **Schritt C** wird die Vorstufe für den Ubergangszustand der chemischen Reaktion ermittelt Dazu wird die optimierte Startgeometrie unter Anwendung eines Monte-Carlo-Algorithmus variiert indem zufällig generierte Auslenkungen von Atomen in einem Ensemble, das aus einem Molekul oder bevorzugt einer Geometrie von mindestens zwei Molekulen I und II besteht, vorgenommen

Monte-Carlo-Algorithmen oder -Verfahren sind Simulationsverfahren, die analytisch nicht oder nur schwer lösbare mathematische Probleme durch nummerische Naherungen losen. Dabei wird ein wahrscheinlichster Verlauf eines Experiments durch eine Vielzahl an zufällig angeordneten Einzelexperimenten beschrieben

In dem erfindungsgemaßen in Schritt C angewandten Monte-Carlo-Algorithmus werden zufällig generierte Auslenkungen von Atomen m einem Ensemble, das aus einem Molekul oder einer Geometrie von mindestens zwei Molekülen I und II besteht, vorgenommen und die Änderung einer Observablen beobachtet. Bei dieser Observablen handelt es sich um eine Gradient-Norm. Dabei variiert das erfindungsgemaße Verfahren nur einen Unterraum der deutlich niedriger dimensional ist als der gesamte Funktionsraum des Ensembles und betrachtet die Anderung der Observablen des gesamten Ensembles als Funktion des reduzierten Funktionsraums

Dazu werden aus dem in Schritt B1 ausgewählten Funktionsraum, in Schritt C1 1 mindestens ein Atom ausgewahlt. Dies geschieht mit Hilfe einer Zufallszahl Z1 Die Richtung einer Auslenkung des durch Z1 gewählten Atoms, bzw die Richtung des Vektors fur die Auslenkung, wird in Schritt C1.2 durch eine weitere Zufallszahl Z2 ausgewahlt Die Richtung wird bevorzugt definiert, indem im bevorzugten Schritt C1 2a die Position eines anderen Atoms als dem in Schritt C1.1 ausgewahlten aus dem reduzierten Funktionsraum ausgewählt wird und dann im bevorzugten Schritt C1 2b die Richtung der Auslenkung ausgewahlt wird, wobei die Position des in Schritt C1 2a ausgewählten Atoms die Richtung des Vektors bestimmt. Die Amplitude der Auslenkung wird bevorzugt durch eine dritte Zufallszahl Z3 im bevorzugten Schritt C1.2c bestimmt.

In einer bevorzugten Ausführungsform des erfindungsgemäßen Verfahrens umfasst Schritt **C1.2** die weiteren folgenden Schritte

C1 2a Bestimmung eines weiteren Atoms aus dem in Schritt B1 ausgewählten Funktionsraum, das nicht mit dem in Schritt C1.1 ausgewählten Atom übereinstimmt,

C1 2b Auswahl der Richtung des Vektors, wobei die Position des in Schritt C1.2.a ausgewahlten Atoms die Richtung des Vektors bestimmt,

C1.2 c zufällige Auswahl der Lange des Vektors, wobei der Wert für die Länge des Vektors positive oder negative Werte annehmen kann.

Dadurch dass die Position des in Schritt C1.2 ausgewählten Atoms die Richtung des Vektors bestimmt, wird der Funktionsraum moglichst stark beschränkt und diese Vorgehensweise führt auch dazu das vorrangig Bindungslangen variiert weiden, die Langen aufweisen wie sie sehr wahrschemlich tatsachlich wahrend einer Reaktion erreicht werden

In Schritt C1 3 wird das in Schritt C 1 1 ausgewählte Atom anhand des Vektors aus Schritt 1 2 aus seiner Position in der optimierten Startgeometrie ausgelenkt, so dass eine Vorstufe für den Ubergangszustand der chemischen Reaktion erhalten wird.

Die erhaltene Vorstufe für den Ubergangszustand wird in Schritt C2 einer Geometrieoptimierung mittels der quantenchemischen Methode unterzogen mit der Randbedingung, dass die mindestens eine Bindung aus Schritt A2 die Lange hat, die in Schritt C1.3 ermittelt wurde. In Schritt C2 wird die gleiche quantenchemische Methode verwendet wie in den Schritten B2 und B3 Dann wird in Schritt C3 die Gradient-Norm C3 für die Vorstufe für den Ubergangszustand aus Schritt C2 ermittelt, wobei die Gradient-Norm durch die erste Ableitung der Funktion E = f(x) mittels der quantenchemischen Methode erhalten wird, mit E=Gesamtenergie der Vorstufe für den Ubergangszustand und x=Kernkoordinaten des Molekuls in der Vorstufe für den Übergangszustand In Schritt C3 wird die gleiche quantenchemische Methode verwendet wie in den Schritten B2, B3 und C2

Die weitere Fortführung des Verfahrens von der Größe der erhaltenen Gradient-Norm C3 ab. Sofern die Gra-

dient-Norm C3 $\nabla$ 0 $\leq$ $\nabla$ $\leq$ 0,07 $E_h$ $a_0^{-1}$ beträgt, dann kann Schritt D1 des Verfahrens mit der Vorstufe für den Übergangszustand durchgeführt werden Dabei steht $E_h$ für die Hartree Energie, wobei 1 $E_h$ = 4 3597 · $10^{-18}$ J, und $a_0$ steht für den Bohr-Radius, wobei 1 $a_0$ = 5 29 · $10^{-11}$ m

Betragt die Gradient-Norm C3 $\nabla$ > 0,07 $E_h$ $a_0^{-1}$, dann werden in Schritt 4 2 die Schritte C1 bis C3 wiederholt bis eine Gradient-Norm C3 $\nabla$ 0 $\leq$ $\nabla$ $\leq$ 0,07 $E_h$ $a_0^{-1}$ erhalten wird Durch die Wiederholung der Schritte C1 bis C3 wird ein iteratives Verfahren dargestellt, mit dem die Gradient-Norm minimiert werden soll. Daher soll als Ausgangspunkt für die Wiederholung des Verfahrens jeweils eine Molekülgeometrie gewahlt werden, die bereits eine moglichst niedrige Gradient-Norm aufweist Bei der Wiederholung der Schritte C1 bis C3 kann also in Schritt C1 nicht nur die optimierte Startgeometrie variiert werden, sondern auch eine Vorstufe für den Ubergangszustand, sofern der Wert ihrer Gradient-Norm C3 kleiner ist als derjenige der optimierten Startgeometrie. Bevorzugt weiden die bei der Durchführung des Verfahrens erhaltene optimierte Startgeometrie und erhaltene(n) Vorstufe(n) für den Ubergangszustand sowie die Werte der jeweiligen Gradient-Normen C3 gesperchert

In dem Fall, dass die Schritte C1 bis C3 ein Mal durchgeführt wurden, kann aus zwei Molekulgeometrien zur Durchführung der Wiederholung diejenige mit der niedrigsten Gradient-Norm ausgewahlt werden, namlich entweder die optimierte Startgeometrie oder die in ersten Durchgang erhaltene Vorstufe für den Ubergangszustand In dem Fall, dass die Schritte C1 bis C3 ein Mal durchgeführt wurden, wird in Schritt C1 die Geometrie der Vorstufe für den Übergangszustand variiert, wenn der Wert ihrer Gradient-Norm C3 niedriger ist als der Wert der Gradient-Norm B3 der optimierten Startgeometrie

In dem Fall, dass die Schritte C1 bis C3 schon durchgeführt wurden, kann aus mehr als zwei Molekülgeometrien zur Durchführung der Wiederholung diejenige mit der medrigsten Gradient-Norm ausgewählt werden, nämlich entweder die optimierte Startgeometrie oder die in bei den Wiederholungen erhaltenen (mehr als eine) Vorstufen für den Übergangszustand. In dem Fall dass die Schritte C1 bis C3 mehr als ein Mal durchgeführt wurden, wird in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist

Bevorzugt ist in Schritt **C4.1** die Gradient-Norm C3 $\leq$ 0,05 $E_h$ $a_0^{-1}$, bevorzugt C3 $\leq$ 0,04 $E_h$ $a_0^{-1}$, bevorzugter $\leq$ 0,03 $E_h$ $a_0^{-1}$, und Schritt **C4.2** wird so lange durchgeführt, bis eine Gradient-Norm C3 $\leq$ 0,05 $E_h$ $a_0^{-1}$, bevorzugt C3 $\leq$ 0,04 $E_h$ $a_0^{-1}$, bevorzugter $\leq$ 0,03 $E_h$ $a_0^{-1}$, erhalten wird.

Bevorzugt wird Schritt **C4.2** höchstens 50 Mal, bevorzugt höchstens 40 Mal, bevorzugter hochstens 30 Mal, wiederholt. Bevorzugt kann bei der Durchführung von Schritt **C4.2** bei der Wiederholung von Schritt **C1** ein anderes Atom ausgewählt werden als bei der vorrangegangenen Durchführung des Verfahrens.

Ein Vorteil des erfindungsgemäßen Verfahrens ist, dass das bei der Variation unter Anwendung eines Monte-Carlo-Algorithmus nur die Atomkoordinaten des ausgewahlten Funktionsraums, also des Unterraums der durch die ausgewählten, an der betrachteten Bindungsdissoziation oder bevorzugt Synthesereaktion beteiligten Atome im Vektorraum aller im Molekül, oder im Molekulensemble, beinhalteten Atomkoordinaten aufgespannt wird Das heißt, der erfindungsgemäße Monte-Carlo-Algorithmus operiert nur in diesem Unterraum, da seine Funktionen nur hierin definiert sind. Damit ist der Unterraum der an der Dissoziation, oder bevorzugten Synthesereaktion, beteiligten Koordinaten der Funktionsraum des Monte-Carlo-Algorithmus. Die Berechnung der Energien und der Gradient-Vektoren (bzw deren Norm) hangen zwar von den gesamten Koordinaten im Molekul ab, jedoch betrachtet der erfindungsgemaße Monte-Carlo-Algorithmus diese nur in Abhangigkeit der Koordinaten des Unterraumes Damit bei dieser Betrachtung der Funktionswert, die zu minimierende Gradient-Norm, eindeutig bleibt, wird vor jeder Betrachtung des Funktionswerts die gesamte Molekulstruktur durch eine Geometrieoptimierung mit Randbedingung(en) relaxiert. Die Randbedingung(en) sind dabei die durch den Monte-Carlo-Algorithmus erzeugten Bindungsabstande

In **Schritt D1** wird die Vorstufe aus Schritt C4 1 oder die Vorstufe aus Schritt B4.1 mittels der quantenchemischen Methode und eines Pseudo-Newton-Raphson-Algorithmus einer relaxiert, so dass der Ubergangszustand erhalten wird. In Schritt D1 wird die gleiche quantenchemische Methode verwendet wie in den Schritten B2, B3, C2 und C3.

Dazu wird bevorzugt zur Uberführung der jeweiligen Molekülgeometrie in einen Stationaren Punkt (G=0) wird eine Geometrieoptimierung vorgenommen, bei der die Gesamtenergie als Funktion der Kernkoordinaten der im Molekül enthaltenen Atome minimiert wird. Um ein lokales Energieminimum zu erhalten wird bevorzugt die Energie nach Gradienten minimiert (zb. Steepest Descent). Um Sattelpunkte erster Ordnung (chemisch als Ubergangszustände interpretierbar) zu erhalten wird ein Newton-Raphson Algorithmus verwendet.

In Schritt D2 wird gegebenenfalls Ermittlung eines Gleichgewichtszustands durch Auslenkung des Ubergangszustands, so dass ein ausgelenkter Übergangszustand erhalten wird und Relaxation des ausgelenkten Übergangszustands mittels der quantenchemischen Methode, so dass ein Gleichgewichtszustand erhalten wird In Schritt D2 wird die gleiche quantenchemische Methode verwendet wie in den Schritten B2, B3, C2, C3 und D1.

Der oben beschriebenen Vorgehensweise in den Schritten C und D liegen folgende grundsätzliche Überlegungen zugrunde· Der Übergangszustand ist charakterisiert durch eine Sattelpunktstruktur des Reaktionspfades mit einem Gradienten der Große Null und einer negativen Krummung entlang eines geometrischen Freiheitsgrades. Sollen die nachst liegenden lokalen Energiemmima zur Sattelpunktstruktur erhalten werden (chemisch interpretierbar als Edukte, Produkte oder Intermediate), kann eine geometrische Störung der Sattelpunkt-Struktur d.h eine Auslenkung der Atome des Molekuls, vorgenommen werden, so dass eine Struktur mit nicht verschwindendem Gradienten erhalten wird Wird diese Molekulstruktur einer Geometrieoptimierung nach Gradienten unterzogen, können damit lokale Minima erhalten werden, die an den Sattelpunkt angrenzen

In den Schritten **B2, B3, C2, C3, D1** und **D2** wird jeweils dieselbe quantenchemische Methode verwendet. Bevorzugt ist die quantenchemische Methode aus Schritten **B2, B3, C2, C3, D1** und **D2** eine semiempirische Methode, Dichte-Funktional-theoretische Methode oder eine Annaherung der Schrödinger-Gleichung, insbesondere sind Dichte-Funktional-theoretische Methoden bevorzugt, wie beispielsweise das TPSS-Dichtefunktional mit einem def2-SVP-Basissatz, so wie standardmäßig im Turbomole-Programmpaket implementiert. In einer bevorzugten Ausführungsform werden die quantenmechanischen Berechnungen mit dem Programmpaket TURBOMOLE durchgeführt Bevorzugt wird zur Berechnung ein Computer mit 16 Core-Prozessoren mit einer Taktfrequenz von 3.20GHz und 25MB Zwischenspercher mit einem RAM Speichel von 128 GB DDR4 2400 rg ECC verwendet.

Bevorzugt ist die chemische Reaktion eine Synthese ausgewählt aus der Gruppe bestehend aus Polymersynthesen, insbesondere Polymethansynthesen, Synthesen von Monomeren für Polymerisationsreaktionen, industriell benötigte Grundchemikalien, Additiven, Tensiden und pharmakologischen Wirkstoffen Insbesondere ist die chemische Reaktion eine Synthese für mit Katalysatoren erhaltene Grundchemikalien oder Edukte für chemische Synthesen Unter Additiven werden allgemein Additive für Kunststoffe wie Weichmacher, Antioxidantien, Stellmittel verstanden, sowie Zusatze für Kraftstoffe, bei deren Synthese jeweils Katalysatoren verwendet weiden.

Wenn bei der bevorzugten Ausführungsform des Verfahrens mindestens zwei Molekule I und II bereitgestellt werden, konnen bevorzugt die unter den Buchstaben **A, B,** und **C** des Verfahrens genannten Schritte wiederholt werden, wobei bei jeder Wiederholung im Vergleich zur von angegangenen Durchführungen des Verfahrens

Molekül I verandert wird oder ein anderes Molekül als Molekul I bereitgestellt wird, und

Molekül II nicht verändert wird und auch kein anderes Molekul als Molekül II bereitgestellt wird,

und der zusätzlichen Schritt D0 durchgeführt werden:

**D0** Vergleich der bei den Wiederholungen erhaltenen Gradienten-Norm C3 für die verschiedenen Vorstufen für den Ubergangszustand und Auswahl der Vorstufe für den Übergangszustand mit der niedrigsten Gradienten-Norm C3 und Durchführung von Schritt D1 und/oder D2 mit der ausgewählten Vorstufe für den Übergangszustand

Bei dieser zuvor beschriebenen bevorzugten Ausführungsform des Verfahrens werden zunächst für unterschiedliche Kombinationen von Molekulen die Gradient-Norm C3 berechnet und die Ergebnisse gespeichert Dann können in emem bevorzugten Schritt D0 die erhaltenen Werte für die Gradient-Norm C3 verglichen werden und die Kombination von Molekulen mit der niedrigsten Gradienten-Norm C3 ausgewählt weiden Diese bevorzugte Ausführungsform des Verfahrens ermoglicht also den direkten Vergleich von unteischiedlichen Kombinationen von Molekülen

Ein weiterer Gegenstand der Erfindung ist ein System zur Datenverarbeitung umfassend Mittel zur Ausführung eines erfindungsgemaßen Verfahrens.

Daruber hinaus ist ein Gegenstand der Erfindung ein Computerprogramm, umfassend Befehle, die bei der Ausführung des Programms durch einen Computer diesen veranlassen, die folgenden Schritte eines Verfahrens durchzufuhren

**Optional A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Molekuls und Auswahl einer Länge der Bindung, wobei die ausgewählte Länge nicht der Lange der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewahlte Lange der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordmaten des Moleküls in der optimierten Startgeometrie,

B4.1 wenn die Gradient-Norm B3 $\nabla$ $0 \leq \nabla \leq 0{,}07\ E_h\ a_0^{-1}$ betragt, dann Einordnung der optimierten Startgeometrie als eine Vorstufe für den Übergangszustand der chemischen Reaktion und Fortführung des Verfahrens mit Schritt D1, oder

B4 2 wenn die Gradient-Norm B3 $\nabla > 0{,}07\ E_h\ a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewahlt wird,
C1.2 ein Vektor für eine Auslenkung des in Schritt C1.1 gewählten Atoms zufällig ausgewählt wird,
C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls m der Vorstufe für den Übergangszustand,

C4.1 wenn die Gradient-Norm C3 $\nabla$ $0 \leq \nabla \leq 0{,}07\ E_h\ a_0^{-1}$ beträgt, dann Fortführung des Verfahrens mit Schritt D1, oder

C4.2 wenn die Gradient-Norm C3 $\nabla > 0{,}07\ E_h\ a_0^{-1}$ beträgt, dann Wiederholung der Schritte C1 bis C3 bis eine Gradient-Norm C3 $0 \leq \nabla \leq 0{,}07\ E_h\ a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Noim B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen varnert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schutt B4.1 mittels der quantenchemischen Methode und eines Pseudo-Newton-Raphson-Algorithmus, so dass der Ubergangszustand erhalten wird,
D2 gegebenenfalls Ermittlung eines Gleichgewichtszustands durch Auslenkung des Ubergangszustands, so dass ein ausgelenkter Ubergangszustand erhalten wird und Relaxation des ausgelenkten Ubergangszustands mittels der quantenchemischen Methode, so dass em Gleichgewichtszustand erhalten wird

Bevorzugt umfasst das Computerprogramm Befehle, die bei der Ausführung des Programms durch einen Computer diesen veranlassen, die Schritte B bis D des Verfahrens durchzuführen Außerdem ist ein weiterer Gegenstand der Erfindung ein computerlesbaies Speichermedium, umfassend Befehle, die bei der Ausführung durch einen Computer diesen veranlassen, die folgenden Schritte eines Verfahrens durchzuführen.

**Optional A Erzeugen einer Startgeometrie**

A1 Bereitstellung der dreidimensionalen Darstellung mindestens eines Moleküls im energetischen Grundzustand,

A2 Auswahl von mindestens einer Bindung des mindestens einen Moleküls und Auswahl einer Lange der Bindung, wobei die ausgewählte Lange nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Lange der mindestens einen Bindung konstant gehalten wird, so dass eine optimierte Startgeometrie erhalten wird,

B3 Ermittlung der Gradient-Norm B3 für die optimierte Startgeometi ie, wobei die Gradient-Norm durch die erste Ableitung einer Funktion $E = f(x)$ mittels der quantenchemischen Methode erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}07$ $E_h$ $a_0^{-1}$ betragt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}07$ $E_h$ $a_0^{-1}$ beträgt, dann Ermittlung der Vorstufe für den Ubergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem m Schritt B1 ausgewählten Funktionsraum zufällig ausgewählt wird,

C1.2 ein Vektor für eine Auslenkung des in Schritt C1.1 gewählten Atoms zufällig ausgewählt wird,

C1.3 das in Schritt C1.1 ausgewahlte Atom anhand des Vektors aus Schritt C1.2 aus seiner Position in der optimierten Startgeometrie ausgelenkt wird, so dass eine Vorstufe für den Ubergangszustand der chemischen Reaktion erhalten wird,

C2 Geometrieoptimierung der Vorstufe für den Ubergangszustand mittels der quantenchemischen Methode und mit der Randbedingung, dass die mindestens eine Bindung aus Schritt A2 die Lange hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion $E = f(x)$ mittels der quantenchemischen Methode erhalten wird, mit E=Gesamtenergie der Vorstufe für den Ubergangszustand und x=Kernkoordinaten des Moleküls in der Vorstufe für den Übergangszustand,

C4.1 wenn die Gradient-Norm C3 $\nabla$ $0 \leq \nabla \leq 0{,}07$ $E_h$ $a_0^{-1}$ betragt, dann Fortführung des Verfahrens mit Schritt D1, oder

C4.2 wenn die Gradient-Norm C3 $\nabla > 0{,}07$ $E_h$ $a_0^{-1}$ betragt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,
D2 gegebenenfalls Ermittlung eines Gleichgewichtszustands durch Auslenkung des Übergangszustands, so dass ein ausgelenkter Übergangszustand erhalten wird und Relaxation des ausgelenkten Übeigangszustands mittels der quantenchemischen Methode, so dass ein Gleichgewichtszustand erhalten wird.

Bevorzugt umfasst das computerlesbare Speichermedium Befehle, die bei der Ausführung des Programms durch einen Computer diesen veranlassen, die Schritte B bis D des Verfahrens durchzuführen

Des Weiteren bezieht sich die Erfindung auf die Verwendung des erfindungsgemäßen Computerprogramms oder des erfindungsgemäßen computerlesbaren Sperchermediums zur Bewertung von Ubergangszustanden einer chemischen Reaktion, insbesondere einer Polymersynthese

Die Erfindung betrifft insbesondere die folgenden Ausführungsformen

Nach einer ersten Ausführungsform betrifft die Erfindung ein computerimplementiertes Verfahren zur Berechnung von Übergangszuständen einer chemischen Reaktion umfassend die Schritte

### A Erzeugen einer Startgeometrie

A1 Bereitstellung der dreidimensionalen Darstellung mindestens eines Moleküls im energetischen Grundzustand,
A2 Auswahl von mindestens einer Bindung des mindestens einen Moleküls und Auswahl einer Lange der Bindung, wobei die ausgewählte Lange nicht der Länge der Bindung im energetischen Grundzustand des Moleküls entspricht, so dass eine Startgeometrie für die chemische Reaktion erhalten wird,
A3 Dreidimensionale Darstellung der Startgeometrie in kartesischen und/oder internen Koordinaten,

### B Ermittlung einer optimierten Startgeometrie

B1 Festlegung eines Funktionsraumes welcher die mindestens eine Bindung aus Schritt A2 und die durch diese Bindung verbundenen Atome umfasst,
B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewahlte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,
B4.1 wenn die Gradient-Norm B3 $\nabla$ $0 \leq \nabla \leq 0{,}07$ $E_h$ $a_0^{-1}$ betragt, dann Einordnung der optimierten Startgeometrie als eine Vorstufe für den Übergangszustand der chemischen Reaktion und Fortführung des Verfahrens mit Schritt D1, oder
B4.2 wenn die Gradient-Norm B3 $\nabla > 0{,}07$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte.

### C Ermittlung der Vorstufe für den Übergangszustand der chemischen Reaktion

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,
C1.2 ein Vektor für eine Auslenkung des in Schritt C1.1 gewählten Atoms zufällig ausgewählt wird,
C1.3 das in Schutt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0,07$ $E_h$ $a_0^{-1}$ betragt, dann Fortführung des Verfahrens mit Schritt D1, oder

C4.2 wenn die Gradient-Norm C3 $\nabla$ > 0,07 $E_h$ $a_0^{-1}$ beträgt, dann Wiederholung der Schritte C1 bis C3 bis eine Gradient-Norm C3 $0 \leq \nabla \leq 0,07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Ubergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Ubergangszustand erhalten wird,

D2 gegebenenfalls Ermittlung eines Gleichgewichtszustands durch Auslenkung des Ubergangszustands, so dass ein ausgelenkter Ubergangszustand erhalten wird und Relaxation des ausgelenkten Ubergangszustands mittels der quantenchemischen Methode, so dass ein Gleichgewichtszustand erhalten wird

In einer zweiten Ausfuhrungsform betrifft die Erfindung ein Verfahren nach Ausführungsform 1, dadurch gekennzeichnet, dass das mindestens eine Molekul aus Schritt A1 eine Große von hochstens 100 Atomen aufweist und/oder dass die Länge der in Schritt **A2** ausgewahlten Bindung höchstens 230 pm beträgt

In einer dritten Ausführungsform betrifft die Erfindung ein Verfahren nach Ausführungsform 1 oder 2, dadurch gekennzeichnet, dass Schritt **C1.2** die weiteren folgenden Schritte umfasst.

C1.2a Bestimmung eines weiteren Atoms aus dem in Schritt B1 ausgewählten Funktionsraum, das nicht mit dem in Schritt C1.1 ausgewählten Atom übereinstimmt,

C1.2b Auswahl der Richtung des Vektors, wobei die Position des in Schritt C1.2.a ausgewählten Atoms die Richtung des Vektors bestimmt,

C1.2.c zufällige Auswahl der Länge des Vektors, wobei der Wert für die Länge des Vektors positive oder negative Werte annehmen kann.

In einer vierten Ausführungsform betrifft die Erfindung ein Verfahren nach einer der vorstehenden Ausführungsformen, dadurch gekennzeichnet, dass in Schritt **C4.1** die Gradient-Norm
C3 $\leq$ 0,05 $E_h$ $a_0^{-1}$, bevorzugt C3 $\leq$ 0,04 $E_h$ $a_0^{-1}$, bevorzugter
$\leq$ 0,03 $E_h$ $a_0^{-1}$, ist und Schritt **C4.2** so lange durchgeführt wird, bis eine Gradient-Norm
C3 $\leq$ 0,05 $E_h$ $a_0^{-1}$ , bevorzugt C3 $\leq$ 0,04 $E_h$ $a_0^{-1}$, bevorzugter $\leq$ 0,03 $E_h$ $a_0^{-1}$, erhalten wird

In einer fünften Ausführungsform betrifft die Erfindung ein Verfahren nach einer der vorstehenden Ausführungsformen, dadurch gekennzeichnet, dass Schritt **C4.2** hochstens 50 Mal, bevorzugt hochstens 30 Mal, wiederholt wird.

In einer sechsten Ausführungsform betrifft die Erfindung ein Verfahren nach einer der vorstehenden Ausführungsformen dadurch gekennzeichnet, dass bei der Durchführung von Schritt **C4.2** bei der Wiederholung von Schritt **C1** ein anderes Atom ausgewahlt werden kann als bei der vorrangegangenen Durchführung des Verfahrens

In einer siebten Ausführungsform betrifft die Erfindung ein Verfahren nach einer der vorstehenden Ausführungsformen, dadurch gekennzeichnet, dass die quantenchemische Methode aus Schritten **B2, B3, C2, C3, D1** und **D2** eine semiempirische Methode, Dichte-Funktional-theoretische Methode oder eine Annaherung der Schrödinger-Gleichung ist, insbesondere ist die quantenchemische Methode aus Schritten **B2, B3, C2, C3, D1** und **D2** eine Dichte-Funktional-theoretische Methode

In einer achten Ausführungsform betrifft die Erfindung ein Verfahren nach einer der vorstehenden Ausführungsformen, dadurch gekennzeichnet, dass die chemische Reaktion eine Synthese ist ausgewählt aus der Gruppe bestehend aus Polymersynthesen, insbesondere Polyurethansynthesen, Synthesen von Monomeren für Polymerisationsreaktionen, industriell benötigte Grundchemikalien, Additiven, Tensiden und pharmakologischen Wirkstoffen.

In einer neunten Ausführungsform betrifft die Erfindung ein Verfahren nach einer der vorstehenden Ausführungsformen, dadurch gekennzeichnet, dass in Schritt **A1** mindestens zwei Moleküle I und II bereitgestellt werden und in Schritt **A2** alternativ oder zusätzlich zu der mindestens einen Bindung auch mindestens ein Abstand zwischen mindestens einem Atom aus Molekul I und mindestens einem Atom aus Molekül II sowie die Länge des mindestens einen Abstands ausgewählt werden können, wobei die Länge des Abstands insbesondere hochstens 230 pm beträgt

In einer zehnten Ausführungsform betrifft die Erfindung ein Verfahren nach Ausführungsform 9, dadurch gekennzeichnet, dass die unter den Buchstaben **A, B,** und **C** des Verfahrens genannten Schritte wiederholt werden, wobei bei jeder Wiederholung im Vergleich zur von angegangenen Durchführungen des Verfahrens

Molekul I verändert wird oder ein anderes Molekul als Molekül I bereitgestellt wird, und

Molekül II nicht verändert wird und auch kein anderes Molekul als Molekül II bereitgestellt wird,

und umfassend einen zusätzlichen Schritt

**D0** Vergleich der bei den Wiederholungen erhaltenen Gradienten-Norm C3 für die verschiedenen Vorstufen für den Übergangszustand und Auswahl der Vorstufe für den Übergangszustand mit der niedrigsten Gradienten-Norm C3 und Durchführung von Schritt D1 und/oder D2 mit der ausgewählten Vorstufe für den Übergangszustand.

In einer elften Ausführungsform betnfft die Erfindung ein Verfahren nach einer der Ausführungsformen 9 oder 10, dadurch gekennzeichnet, dass Molekül I ein Katalysator für die chemische Reaktion ist, insbesondere für eine Polymersynthese, und Molekul II ein Edukt der chemischen Reaktion

In einer zwölften Ausführungsform betrifft die Erfindung ein Verfahren nach einer der Ausführungsformen 9 bis 11, dadurch gekennzeichnet, dass die Lange des m Schritt **A2** ausgewahlten Abstands zwischen mindestens emem Atom aus Molekül I und/oder mindestens einem Atom aus Molekul II insbesondere hochstens 230 pm beträgt.

In einer dreizehnten Ausführungsform betrifft die Erfindung ein Verfahren nach einer der Ausführungsformen 9 bis 12, dadurch gekennzeichnet, dass Molekül I eine Große von $\leq$ 100 Atomen aufweist und Molekül II eine Größe von $\leq$ 100 Atomen aufweist, bevorzugt soll die Summe der Atome aus Molekül I und aus Molekul II $\leq$ 100 Atome betragen.

In einer vierzehnten Ausführungsform betrifft die Erfindung ein System zur Datenverarbeitung umfassend Mittel zur Ausführung eines Verfahrens umfassend die Schritte.

## A Erzeugen einer Startgeometrie

A1 Bereitstellung der dreidimensionalen Darstellung mindestens eines Molekuls im energetischen Grundzustand,

A2 Auswahl von mindestens einer Bindung des mindestens einen Molekuls und Auswahl einer Lange der Bindung, wobei die ausgewahlte Länge nicht der Länge der Bindung im energetischen Grundzustand des Molekuls entspricht, so dass eine Startgeometrie für die chemische Reaktion erhalten wird,

A3 Dreidimensionale Darstellung der Startgeometrie in kartesischen und/oder internen Koordinaten,

## B Ermittlung einer optimierten Startgeometrie

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewahlten Funktionsraums mittels einer quantenchemischen Methode und mit der Randbedingung, dass die in Schritt A2 ausgewählte Lange der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Molekuls in der optimierten Startgeometrie,

B4.1 wenn die Gradient-Norm B3 $\nabla$ $0 \leq \nabla \leq 0,07$ $E_h$ $a_0^{-1}$ betragt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0,07$ $E_h$ $a_0^{-1}$ beträgt, dann Ermittlung der Vorstufe für den Ubergangszustand der chemischen Reaktion ausgehend von der optimierten Startgeometrie durch ein Verfahren umfassend die folgenden Schritte

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,
C1.2 ein Vektor für eine Auslenkung des in Schritt C1.1 gewählten Atoms zufällig ausgewählt wird,
C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Übergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder

C4.2 wenn die Gradient-Norm C3 $\nabla > 0{,}07$ $E_h$ $a_0^{-1}$ beträgt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,
D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird

In einer fünfzehnten Ausführungsform betrifft die Erfindung ein Computerprogramm, umfassend Befehle, die bei der Ausführung des Programms durch einen Computer diesen veranlassen, die folgenden Schritte eines Verfahrens durchzufuhren

**Optional A Erzeugen einer Startgeometrie**

A1 Bereitstellung der dreidimensionalen Darstellung mindestens eines Moleküls im energetischen Grundzustand,
A2 Auswahl von mindestens einer Bindung des mindestens einen Moleküls und Auswahl einer Lange der Bindung, wobei die ausgewählte Länge nicht der Länge der Bindung im energetischen Grundzustand des Moleküls entspricht, so dass eine Startgeometrie für die chemische Reaktion erhalten wird,
A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,
B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}07$ $E_h$ $a_0^{-1}$ beträgt, dann Einordnung der optimierten Startgeometrie als eine Vorstufe für den Übergangszustand der chemischen Reaktion und Fortführung des Verfahrens mit Schritt D1, oder

B4.2 wenn die Gradient-Norm B3 $\nabla > 0{,}07$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte.

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewählten Funktionsraum zufällig ausgewählt wird,

C1.2 ein Vektor für eine Auslenkung des in Schritt C1.1 gewählten Atoms zufällig ausgewählt wird,

C1.3 das in Schritt C1.1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Ubergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Übergangszustand,

C4.1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder

C4 2 wenn die Gradient-Norm C3 $\nabla > 0{,}07$ $E_h$ $a_0^{-1}$ betragt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b)für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Ubergangszustand erhalten wird,

D2 gegebenenfalls Ermittlung eines Gleichgewichtszustands durch Auslenkung des Ubergangszustands, so dass ein ausgelenkter Übergangszustand erhalten wird und Relaxation des ausgelenkten Ubergangszustands mittels der quantenchemischen Methode, so dass ein Gleichgewichtszustand erhalten wird

In einer sechzehnten Ausführungsform betrifft die Erfindung ein computerlesbares Speichermedium, umfassend Befehle, die bei der Ausführung durch einen Computer diesen veranlassen, die folgenden Schritte eines Verfahrens durchzuführen

**Optional A Erzeugen einer Startgeometrie**

A1 Bereitstellung der dreidimensionalen Darstellung mindestens eines Molekuls im energetischen Grundzustand,

A2 Auswahl von mindestens einer Bindung des mindestens einen Molekuls und Auswahl einer Lange der Bindung, wobei die ausgewählte Länge nicht der Länge der Bindung im energetischen Grundzustand des Moleküls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewahlten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Molekuls in der optimierten Startgeometrie,

B4.1 wenn die Gradient-Norm B3 $\nabla$ $0 \leq \nabla \leq 0{,}07$ $E_h$ $a_0^{-1}$ betragt, dann Einordnung der optimierten Startgeometrie als eine Vorstufe für den Ubergangszustand der chemischen Reaktion und Fortführung des Verfahrens mit Schritt D1, oder

B4 2 wenn die Gradient-Norm B3 $\nabla > 0{,}07$ $E_h$ $a_0^{-1}$ beträgt, dann Ermittlung der Vorstufe für den Übergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1 1 wenigstens ein Atom aus dem in Schritt B1 ausgewahlten Funktionsraum zufällig ausgewählt wird,

C1.2 ein Vektor für eine Auslenkung des in Schritt C1 1 gewählten Atoms zufällig ausgewahlt wird,

C1 3 das in Schritt C1 1 ausgewählte Atom anhand des Vektors aus Schritt C1.2 aus seiner Position in der optimierten Startgeometrie ausgelenkt wird, so dass eine Vorstufe für den Ubergangszustand der chemischen Reaktion erhalten wird,

C2 Geometrieoptimierung der Vorstufe für den Ubergangszustand mittels der quantenchemischen Methode und mit der Randbedingung, dass die mindestens eine Bindung aus Schritt A2 die Lange hat, die in der Schritt C1 3 ermittelt wurde,

C3 Ermittlung der Gradient-Norm C3 für die Vorstufe für den Ubergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Molekuls in der Vorstufe für den Ubergangszustand,

C4.1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder

C4 2 wenn die Gradient-Norm C3 $\nabla > 0{,}07$ $E_h$ $a_0^{-1}$ beträgt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Ubergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Ubergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,
D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird

In einer siebzehnten Ausführungsform betrifft die Erfindung ein Verwendung eines Computerprogramms nach Ausführungsform 15 oder eines computerlesbaren Speichermediums nach Ausführungsform 18 zur Bewertung von Ubergangszuständen einer chemischen Reaktion, insbesondere einer Polymersynthese.

Anhand der nachfolgenden Beispiele soll die Erfindung veideutlicht werden, ohne jedoch darauf beschränkt zu werden

**A Beispiele zur Ermittlung einer voroptimierten Startgeometrie**

Das erfindungsgemäße Verfahren und ein herkömmliches Verfahren wurden auf verschiedene Reaktionen angewandt Dazu wurde zunächst für jede Reaktion mit Hilfe eines kommerziell erhaltlichen Programms zur Generierung und Visualisierung von dreidimensionalen Strukturen chemischer Molekule eine Startgeometrie des unbekannten, gesuchten Ubergangszustandes gezeichnet Dabei wurden die Abstände zwischen den Atomen bzw der Bindungen geschätzt

In Vergleichsversuchen wurde diese Startgeometrie einer Geometrieoptimierung mit einer Quasi-Newton-Raphson-Methode unterzogen, wie sie im Stand der Technik verwendet wird. Hierzu wurden zunächst die an der Bindungsdissoziation beteiligten Bindungsabstände konstant gehalten und eine Voroptimierung durchgeführt. Anschließend wurde die Quasi-Newton-Raphson-Methode angewendet Dabei konnte kein Ubergangszustand lokalisiert werden. Eine Wiederholung des letzten Schritts bringt keine Veranderung des Ergebnisses, da es sich um eine deterministische Methode handelt

In den erfindungsgemäßen Versuchen wurde auf dieselbe Startgeometrie das erfindungsgemäße Verfahren angewendet. Dazu wurde zunächst die Voroptimierung mit der erfindungsgemäßen Monte-Carlo-Methode vorgenommen Die erfindungsgemäße Monte Carlo-Methode variiert die festgelegten Bindungslangen mit Dissoziationscharakter nach Zufallszahlen und führt anschließend Geometrieoptimierungen mit konstant gehaltenen Bindungsabständen durch Diese Prozedur wird iterativ durchlaufen, bis die erhaltene Gradient-Norm unter einen festgelegten Wert minimiert ist, wodurch eine ausreichende Nähe zu einem stationären Punkt indiziert wird. Pro untersuchter Reaktion wurden mit dem erfindungsgemäßen Monte-Carlo-Verfahren 10 optimierte Startgeometrien mit dem jeweils gleichen Wert für die Gradient-Norm als Ausgangspunkt für die anschließende Geometrieoptimierung mittels Quasi-Newton-Raphson gewahlt

Die Berechnungen und die Erfolgsquoten, d h die Anzahl der Übergangszustande, die auf einer Grundlage von jeweils zehn optimierten Startgeometrien ermittelt werden konnte, sind in Tabellen A1 bis A5 dargestellt

Alle quantenmechanischen Berechnungen wurden dabei mit dem Programmpaket TURBOMOLE durchgeführt Die verwendete Dichtefunktionaltheoretische Methode war das TPSS-Dichtefunktional mit einem def2-SVP-Basissatz, so wie standardmaßig im Turbomole-Programmpaket implementiert. Dazu wurde als Computer ein Intel Xeon E5-2667v4 mit 16 Core-Prozessoren mit einer Taktfrequenz von 3.20GHz und 25MB Zwischenspeicher mit einem RAM Speicher von 128 GB DDR4 2400 rg ECC.

Unter Verwendung dieses Rechners konnten mit dem erfindungsgemäßen Verfahren innerhalb von hochstens 2 Stunden für 10 unterschiedliche, manuell ausgewählte Startgeometrien einer Reaktion verschiedene Ubergangszustände für diese Reaktion berechnet werden Dabei konnte das Verfahren von dem Rechner auch ohne Kontrolle durch einen Mitarbeiter, z.B uber Nacht, durchgeführt werden

Mit herkömmlichen Verfahren, bei denen die Startgeometrie manuell, d. h. ohne ein computerimplementiertes Verfahren, variiert wird, ware ein Zeitaufwand von 2 bis 3 Tagen pro Startgeometrie erforderlich bis eine Geometrie gefunden worden ware, auf die ein Pseudo-Newton-Raphson-Verfahren angewendet weiden kann, d h. erst nach 2 bis 3 könnte uberhaupt versucht werden einen Ubergangszustand zu berechnen

In der nachfolgenden Tabelle A 1 ist zusammengefasst, wie oft Schritt B, bzw die Schritte B und C, durchgeführt wurden

- für alle optimierten Startgeometrien pro untersuchter Reaktion, bis eine Vorstufe für den Übergangszustand erhalten wurde oder das Verfahren abgebrochen wurde, weil keine Vorstufe für den Übergangszustand erhalten wurde (Zeile 1), oder

- für diejenigen optimierten Startgeometrien, für die ein Ubergangszustand ermittelt werden konnte (Zeile 2).

**Tabelle A.1: Statistische Auswertung**

| Zeile | Beispiele | Tab. 2 | Tab. 3 | Tab. 4 | Tab. 5 |
|---|---|---|---|---|---|
| 1 | Durchschnittliche Anzahl der Durchlaufe von Schritt C des erfindungsgemäßen Verfahrens für alle optimierten Startgeometrien einer Reaktion | 6,6 | 9,7 | 8,2 | 9,2 |
| 2 | Durchschnittliche Anzahl der Durchläufe von Schritt C des erfindungsgemäßen Verfahrens für diejenigen optimierten Startgeometrien, für die ein Übergangszustand ermittelt werden konnte. | 5,5 | 12,3 | 8,2 | 9,2 |
| 3 | Prozent der jeweiligen optimierten Startgeometrien eines Versuchs für die mit dem erfindungsgemäßen Verfahren ein Übergangszustand ermittelt werden konnte | 80 | 30 | 100 | 100 |

Für die Bromierung von Ethen, in der folgenden Tabelle A3 dargestellt ist es relativ schwer einen Übergangszustand zu berechnen Dies liegt sehr wahrscheinlich daran, dass der Sattelpunkt eine sehr spitze Form aufweist.

**Tabelle A.2: Cycloaddition von 1,3-Butadien und Ethen**

| | Jede Spalte zeigt jeweils eine andere optimierte Startgeometrie | | | | | | | | | |
|---|---|---|---|---|---|---|---|---|---|---|
| Beispiel | 2 1 | 2 2 | 2 3 | 2 4 | 2 5 | 2 6 | 2 7 | 2 8 | 2 9 | 2 10 |
| Gradient-Norm B3 [$E_h$ $a_0^{-1}$] der jeweiligen optimierten Startgeometrie | 0 101578 | 0 101578 | 0 101578 | 0 101578 | 0 101578 | 0 101578 | 0.101578 | 0 101578 | 0 101578 | 0 101578 |
| Gradient-Norm C3 [$E_h$ $a_0^{-1}$], wobei jede Zeile die Gradient Norm einer anderen Vorstufe für den Ubergangszustand jeweils basierend auf der optimierten Startgeometrie der ersten Zeile darstellt | 0 063475 | 0 112442 | 0 027745 | 0 094052 | 0 125426 | 0 086226 | 0 081093 | 0.107781 | 0.087146 | 0 125814 |
| | 0 074802 | 0 074597 | | 0 029719 | 0 091124 | 0 088933 | 0 095923 | 0 111804 | 0 159795 | 0 041008 |
| | 0 357504 | 0 090029 | | | 0 065701 | 0 009156 | 0 206321 | 0 070290 | 0 110302 | 0.667969 |
| | 0 090818 | 0 390142 | | | 0 053928 | | 0 063935 | 0 072940 | 0 118519 | 0 194443 |
| | 0 111428 | 0.092513 | | | 0 012537 | | 0 012706 | 0 056430 | 2 170 497 | 0 013739 |
| | 1 404 968 | 0 067496 | | | | | | 0 061099 | 0.071430 | |
| | 0 111726 | 0 029182 | | | | | | 0 025372 | 0 085191 | |
| | 0 102065 | | | | | | | | 0 024142 | |
| | 0 077888 | | | | | | | | | |
| | 0 085817 | | | | | | | | | |
| | 0 057883 | | | | | | | | | |
| | 0 194193 | | | | | | | | | |
| | 0 037484 | | | | | | | | | |
| Erhalt eines Übergangszustands | Nein | Nein | Ja | Ja | Ja | Ja | Ja | Ja | Ja | Ja |

EP 3 640 947 A1

In Beispiel 2 1 wurde die Wiederholung des Schritts C des erfindungsgemäßen Verfahrens nach 13 Durchläufen abgebrochen, weil keine Vorstufe für den Übergangszustand ermittelt werden konnte, die als Ausgangspunkt für die Ermittlung eines Übergangszustands mit Hilfe eines Pseudo-Newton-Raphson-Algorithmus nach Schritt D1 geeignet war.

**Tabelle A.3: Bromierung von Ethen zu 1,2 Bromethan**

| Beispiel | 3 1 | 3 2 | 3 3 | 3 4 | 3 5 | 3 6 | 3 7 | 3 8 | 3 9 | 3 10 |
|---|---|---|---|---|---|---|---|---|---|---|
| | Jede Spalte zeigt jeweils eine andere optimierte Startgeometrie | | | | | | | | | |
| Gradient-Norm B3 $[E_h\,a_0^{-1}]$ der jeweiligen optimierten Startgeometrie | 0 056813 | 0 056813 | 0 056813 | 0 056813 | 0.056813 | 0 056813 | 0 056813 | 0 056813 | 0 056813 | 0 056813 |
| Gradient-Norm C3 $[E_h\,a_0^{-1}]$, wobei jede Zeile die Gradient Norm einer anderen Vorstufe für den Ubergangszustand jeweils basierend auf der optimierten Startgeometrie der ersten Zeile darstellt | 0 058529 | 0 064647 | 0 064082 | 0 048314 | 0 031738 | 0 058399 | 0 051681 | 0 058077 | 0 127864 | 0 231416 |
| | 0 251325 | 0 061090 | 0 152230 | 0 137252 | | 0 059887 | 0 044425 | 0 060593 | 0 232556 | 0 056812 |
| | 0 064038 | 0 107419 | 0 051544 | 0 056970 | | 0 060656 | 0 066049 | 0 053602 | 0 057414 | 0 063343 |
| | 0 056935 | 0 065046 | 0 099173 | 0 048362 | | 0 284270 | 0 081247 | 0 070952 | 0 044737 | 0 065012 |
| | 0 059306 | 0 062879 | 0 112987 | 0 056837 | | 0 058757 | 0 057149 | 0 099214 | 0 035028 | 0 256491 |
| | 0 030828 | 0 442817 | 0 050282 | 0 135475 | | 0.060478 | 0 036297 | 0 055646 | | 0 045294 |
| | | 0 058038 | 0.070672 | 0.063971 | | 0.062872 | | 0 046495 | | 0.056140 |
| | | 0 054012 | 0 061423 | 0.045588 | | 0 049047 | | 0 046513 | | 0 045318 |
| | | 0.033991 | | 0 090480 | | 0 054034 | | 0 050048 | | 0 055720 |
| | | | | 0 067227 | | 0 046588 | | 0 048978 | | 0 168430 |
| | | | | 0 044884 | | 0 055280 | | 0 048104 | | 0 059298 |
| | | | | 0.057821 | | | | 0 056875 | | 8 418 276 |
| | | | | | | | | 0 089174 | | |

(fortgesetzt)

| Beispiel | Jede Spalte zeigt jeweils eine andere optimierte Startgeometrie | | | | | | | | | |
|---|---|---|---|---|---|---|---|---|---|---|
| | 3 1 | 3 2 | 3 3 | 3 4 | 3 5 | 3 6 | 3 7 | 3 8 | 3 9 | 3 10 |
| | | | | | | | | 0 057857 | | |
| | | | | | | | | 2 128 698 | | |
| | | | | | | | | 0 085683 | | |
| | | | | | | | | 0 037714 | | |
| Erhalt eines Ubergangszustands | Ja | Nem | Nein | Nein | Nein | Ja | Nein | Ja | Nein | Nein |
| Die gezeigten Werte umfassen auch statistische Extremalwerte, z.B. in Spalten 3 8 und 3 10. | | | | | | | | | | |

**Tabelle A.4: Decarboxylierung von Acetessigsäure**

| Beispiel | Jede Spalte zeigt jeweils eine andere optimierte Startgeometrie | | | | | | | | | |
|---|---|---|---|---|---|---|---|---|---|---|
| | 4 1 | 4 2 | 4 3 | 4 4 | 4 5 | 4 6 | 4 7 | 4 8 | 4 9 | 4 10 |
| Gradient-Norm B3 [$E_h\,a_0^{-1}$] der jeweiligen optimierten Startgeometrie | 0.055244 | 0.055145 | 0 055285 | 0 055128 | 0 055106 | 0 055109 | 0 055218 | 0 055265 | 0 055413 | 0.055211 |
| Gradient-Norm C3 [$E_h\,a_0^{-1}$], wobei jede Zeile die Gradient Norm einer anderen Vorstufe fur den Ubergangszustand jeweils basierend auf der optimierten Startgeometrie der ersten Zeile darstellt | 0 049610 | 0.050850 | 0 057335 | 0 077653 | 0 060983 | 0 047224 | 0.050552 | 0 057350 | 0 058160 | 0 069370 |
| | 0 049458 | 0.062125 | 0 080891 | 0 058727 | 0 065141 | 0 067408 | 0 057388 | 0 621287 | 0 022109 | 0 082815 |
| | 0 019246 | 0 039781 | 0 045796 | 0 043377 | 0 059457 | 0 112063 | 0 034994 | 0 052275 | | 0 087075 |
| | | | 0 053588 | 0 071861 | 0 053003 | 0 051080 | | 0 056791 | | 0 113501 |
| | | | 0 066665 | 0 069440 | 0 051556 | 0 055590 | | 0.072648 | | 0 051053 |
| | | | 0 020932 | 0 077257 | 0 062801 | 0 073973 | | 0 045846 | | 0 048086 |
| | | | | 0 045192 | 0 047389 | 0 087384 | | 0 047361 | | 0 051334 |
| | | | | 0 053762 | 0 113882 | 0 066149 | | 0 015354 | | 0.010003 |
| | | | | 0 057251 | 0 069916 | 0.056958 | | | | |
| | | | | 0 089196 | 0.061307 | 0 083602 | | | | |
| | | | | 0 022387 | 0.045287 | 0 044368 | | | | |
| | | | | | 0 099284 | 0 042818 | | | | |
| | | | | | 0 326467 | 0 054379 | | | | |
| | | | | | 0 053152 | 0 030123 | | | | |
| Erhalt eines Ubergangszustands | Ja | Ja | Ja | Ja | Ja | Ja | Ja | Ja | Ja | Ja |

**Tabelle A.5: Reduktion von Aceton zu Isobuten**

| Beispiel | Jede Spalte zeigt jeweils eine andere optimierte Startgeometrie | | | | | | | | | |
|---|---|---|---|---|---|---|---|---|---|---|
| | 5 1 | 5 2 | 5 3 | 5 4 | 5 5 | 5 6 | 5 7 | 5 8 | 5 9 | 5 10 |
| Gradient-Norm B3 $[E_h\, a_0^{-1}]$ der jeweiligen optimierten Startgeometrie | 0,070525 | 0,070525 | 0,070525 | 0,070525 | 0,070525 | 0,070525 | 0,070525 | 0,070525 | 0,070525 | 0,070525 |
| Gradient-Norm C3 $[E_h\, a_0^{-1}]$, wobei jede Zeile die Gradient Norm einer anderen Vorstufe für den Ubergangszustand jeweils basierend auf der optimierten Startgeometrie der ersten Zeile darstellt | 0,053417 | 0,039139 | 0,080632 | 0,051345 | 0,066935 | 0,067211 | 0,07307 | 0,070434 | 0,070324 | 0,086278 |
| | 0,032317 | | 0,086216 | 0,090356 | 0,047545 | 0,068587 | 0,071768 | 0,064323 | 0,071679 | 0,057452 |
| | | | 0,055797 | 0,054938 | 0,026039 | 0,063053 | 0,054758 | 0,066577 | 0,052235 | 0,019717 |
| | | | 0,065289 | 0,0546 | | 0,056315 | 0,059952 | 0,062511 | 0,061696 | |
| | | | 0,052484 | 0,058829 | | 0,048515 | 0,061317 | 0,060599 | 0,036104 | |
| | | | 0,043561 | 0,054373 | | 0,045782 | 0,069163 | 0,171984 | | |
| | | | 0,057211 | 0,0659 | | 0,031459 | 0,05429 | 0,060208 | | |
| | | | 0,087309 | 0,044661 | | | 0,044025 | 0,066551 | | |
| | | | 0,044804 | 0,283666 | | | 0,044025 | 0,067845 | | |
| | | | 0,053187 | 0,054703 | | | 0,045235 | 0,057384 | | |
| | | | 0,045268 | 0,043981 | | | 0,044025 | 0,06995 | | |
| | | | 0,057168 | 0,039904 | | | | 0,065572 | | |
| | | | 0,053825 | | | | | 0,060108 | | |
| | | | 0,056278 | | | | | 0,060921 | | |
| | | | 0,084044 | | | | | 0,060597 | | |
| | | | 0,043251 | | | | | 0,045019 | | |
| | | | 0,043252 | | | | | 0,057638 | | |
| | | | 0,045823 | | | | | 0,065807 | | |
| | | | 0,039945 | | | | | 0,033495 | | |
| Erhalt eines Ubergangszustands | Ja | Ja | Ja | Ja | Ja | Ja | Ja | Ja | Ja | Ja |

EP 3 640 947 A1

24

**B Beispiele zur Ermittlung eines Katalysators für eine chemische Reaktion**

In den folgenden Beispielen wurde mit Hilfe des erfindungsgemäßen Verfahrens untersucht, welcher der bekannten Katalysatoren 1,4-Diazabicyclo[2 2 2]octan (DABCO) oder N,N-Dimethylannillin (DMA) sich besser für die Hydrolyse von unterschiedlichen Isocyanaten eignet Mit dieser Reaktion kann bei der Herstellung von Polyurethanschaumstoffen die Reaktionsmischung durch den Eintrag des entstandenen Gases aufgetrieben werden, so dass ein Schaum erhalten wird.

In den Beispielen wurde zunächst mit Hilfe des erfindungsgemäßen Verfahrens die Aktivierungsenergien für die betreffende Reaktion von Isocyanat und Wasser berechnet und die erhaltenen Werte dann mit den Ergebnissen von Laborversuchen der zuvor berechneten Reaktionen verglichen Als Maßstab für die Aktivität der getesteten Katalysatoren wurde bei den Laborversuchen die Reaktionstemperatur herangezogen oberhalb derer die Katalysatoren Aktivität zeigten. Ob die Katalysatoren Aktivität zeigten wurde im Laboiexperiment anhand der Freisetzung von Gasen und dem Aufschaumen der Reaktionsmischung festgestellt

Die quantenmechanischen Berechnungen wurden mit dem Programmpaket Turbomole durchgeführt. Die verwendete Dichtefunktionaltheoretische Methode war das TPSS-Dichtefunktional mit einem def2-SVP-Basissatz, so wie standardmaßig im Turbomole-Programmpaket implementiert Dazu wurde als Computer ein Intel Xeon E5-2667v4 mit 16 Core-Prozessoren mit einer Taktfrequenz von 3.20GHz und 25MB Zwischenspeicher mit emem RAM Speicher von 128 GB DDR4 2400 rg ECC.

Es wurden pro untersuchter Reaktion eine Startgeometrie als Ausgangspunkt für die Ermittlung eines Ubergangszustandes gewahlt Der Zeitaufwand für die Berechnung der jeweiligen Übergangszustande betrug bis zu 8 Stunden

Die Laborversuche wurden wie folgt durchgefuhrt·

In emem Reagenzglas mit Magnetruhrer wurden 0,1 Aquivalente Katalysator, 1,0 Aquivalente Polyethylenglykol und 1,0 Äquivalente Wasser vorgelegt. Der Inhalt des Reagenzglas wurde grundlich durchmischt und unter Ruhren 1,0 Äquivalente des betreffenden Isocyanats zugesetzt. Die Temperatur des Gemischs wurde mit Hilfe eines Olbads mit Thermostat langsam erhoht, bis eine deutliche Gasentwicklung beobachtet werden konnte

**Tabelle B.1: Molmassen und Stoffmengen der eingesetzten Substanzen.**

| Komponente | M [g/mol] | n [mol] | m [mg] | Aq | d [g/ml] | V [ml] |
|---|---|---|---|---|---|---|
| DABCO | 112,17 | 1,722 | 193 | 0,1 | - | - |
| DMA | 121,19 | 1,722 | 208 | 0,1 | 0,96 | 0,22 |
| TDI | 147,20 | 1,722 | 3000 | 1,0 | 1,22 | 2,46 |
| IPDI | 222,29 | 1,722 | 3828 | 1,0 | 1,06 | 3,61 |
| $H_2O$ | 18,02 | 1,722 | 310 | 1,0 | 1,00 | 0,31 |
| PEG | 400 | 0,861 | 3444 | 0,5 | 1,128 | 3,05 |

**Beispiel B.1: 1,4-Diazabicyclo[2.2.2]octan vs N,N-Dimethylannillin als Katalysatoren für die Hydrolyse von 1,4-Toluyldiisocyanat**

Zur Evaluierung der katalytischen Aktivität von 1,4-Diazabicyclo[2 2 2]octan zur Katalysierung der Hydrolyse von 1,4-Toluyldiisocyanat mit Wasser wurde die Anlagerung von Wasser an das Isocyanat-Molekül mit der erfindungsgemäßen Methode zur Berechnung von Übergangszustanden berechnet Fur die mit dem erfindungsgemäßen Verfahren ermittelte Geometrie des Ubergangszustands wurde eine Aktivierungsenergie von 12,8 Kcal/mol berechnet Dieselbe Prozedur wurde für N,N-Dimethylannillin (s. Tabelle B 2) wiederholt und eine Aktivierungsenergie von 17 1 Kcal/mol berechnet.

Unter den oben beschriebenen experimentellen Bedingungen zeigte die Umsetzung von 1,4-Toluyldiisocyanat mit Wasser in Gegenwart von 1,4-Diazabicyclo[2.2.2]octan als Katalysator eine signifikante Reaktion bei 20°C Unter identischen Bedingungen wurde N,N-Dimethylannillin als Katalysator getestet Hier war eine Reaktionstemperatur von 39°C notwendig um eine beobachtbare chemische Reaktion zu herbei zu führen.

**Tabelle B.2: Zusammenstellung experimenteller und simulierter Daten**

| Isocyanat | Katalysator | Aktivierungsenergie Simuliert [Kcal/mol) | Temperatur bei der eine Aktivität des Katalysators im Laborversuch zu beobachten war [°C] | Gradientnorm der optimierten Startgeometrie $[E_h\ a_0^{-1}]$ | Gradientnorm des Übergangszustands $[E_h\ a_0^{-1}]$ |
|---|---|---|---|---|---|
| TDI | DABCO | 12,8 | 20 | 0,067 | 0,042 |
| TDI | DMA | 17,1 | 39 | 0,068 | 0,030 |

Dieser Vergleich zeigt, dass mit Hilfe der erfindungsgemäßen Methode die Molekülgeometrie eines Übergangszustands einer chemischen Reaktion so prazise berechnet weiden kann, dass die Aktivierungsenergie, die für diese Molekulgeometrie berechnet wird, eine zutreffende Aussage über die Kinetik der chemischen Reaktion erlaubt Die auf der Grundlage des erfindungsgemäßen Verfahrens berechnete Aktivierungsenergie für DABCO war deutlich niedriger als diejenige für DMA Aus den berechneten Ergebnissen kann der Schluss gezogen werden, dass DABCO als Katalysator für die Hydrolyse von 1,4-Toluyldiisocyanat einen Ubergangszustand ermöglicht der eine niedrigere Energie aufweist als bei der Verwendung von DMA als Katalysator und dass folglich bei der Durchführung der Reaktion mit DABCO als Katalysator weniger oder keine Energie (ausgehend von Raumtemperatur) zugeführt werden muss, damit die Reaktion abläuft und Produkte erhalten werden.

Diese Schlusse aus den Berechnungen spiegeln sich in den Ergebnissen der Laborversuche wieder Wahrend die Hydrolyse von 1,4-Toluyldiisocyanat mit DABCO als Katalysator bereits bei einer Reaktionstemperatur von 20 °C ablief, musste bei der Verwendung von DMA Energie zugeführt werden, und die Reaktion lief erst bei 39 °C ab

## Beispiel B.2: 1,4-Diazabicyclo[2.2.2]octan vs N,N-Dimethylannillin als Katalysatoren für die Hydrolyse von Isophorondiisocyanat

Zur Evaluierung der Katalytischen Aktivität von 1,4-Diazabicyclo[2 2 2]octan zur Katalysierung der Hydrolyse von Isophorondiisocyanat mit Wasser wurde die Anlagerung von Wasser an das Isocyanat-Molekül mit der erfindungsgemäßen Methode zur Berechnung von Ubergangszuständen berechnet Für die mit dem erfindungsgemäßen Verfahren ermittelte Geometrie des Übergangszustands wurde eine Aktivierungsenergie von 16,3 Kcal/mol berechnet. Die gleiche Prozedur wurde für N,N-Dimethylannillin (s Tabelle B 3) wiederholt und eine Aktivierungsenergie von 19,9 Kcal/mol berechnet

Unter den beschriebenen experimentellen Bedingungen zeigte die Umsetzung von Isophorondiisocyanat mit Wasser in Gegenwart von 1,4-Diazabicyclo[2.2.2]octan als Katalysator eine signifikante Reaktion bei 31°C. Unter identischen Bedingungen wurde N,N-Dimethylannillin als Katalysator getestet. Hier war eine Reaktionstemperatur von 116°C notwendig um eine beobachtbare chemische Reaktion zu herbei zu führen.

**Tabelle B.3: Zusammenstellung experimenteller und simulierter Daten**

| Isocyanat | Katalysator | Aktivierungsenergie Simuliert [Kcal/mol] | Temperatur bei der eine Aktivitat des Katalysators im Laborversuch zu beobachten war[°C] | Gradientnorm der optimierten Startgeometrie $[E_h\ a_0^{-1}]$ | Gradientnorm des Ubergangszustands $[E_h\ a_0^{-1}]$ |
|---|---|---|---|---|---|
| IPDI | DABCO | 16,3 | 31 | 0,067 | 0,043 |
| IPDI | DMA | 19,9 | 116 | 0,067 | 0,045 |

Dieser Vergleich zeigt, dass mit Hilfe der erfindungsgemäßen Methode die Molekulgeometne eines Übergangszustands einer chemischen Reaktion mit sehr wenig Benutzeraufwand und so prazise berechnet werden kann, dass die Aktivierungsenergie, die für diese Molekulgeometrie berechnet wird, eine zutreffende Aussage über die Kinetik der chemischen Reaktion erlaubt Die auf der Grundlage des erfindungsgemäßen Verfahrens berechnete Aktivierungsenergie für DABCO war deutlich niedriger als diejenige für DMA Aus den berechneten Ergebnissen kann der Schluss gezogen werden, dass DABCO als Katalysator für die Hydrolyse von 1,4-Toluyldiisocyanat einen Ubergangszustand ermöglicht der eine niedrigere Energie aufweist als bei der Verwendung von DMA als Katalysator und dass folglich bei der Durchführung der Reaktion mit DABCO als Katalysator weniger oder keine Energie (ausgehend von Raumtemperatur) zugeführt werden muss, damit die Reaktion abläuft und Produkte erhalten werden

Diese Schlüsse aus den Berechnungen spiegeln sich in den Ergebnissen der Laborversuche wieder Wahrend die Hydrolyse von 1,4-Toluyldiisocyanat mit DABCO als Katalysator bereits bei einer Reaktionstemperatur bei 31 °C ablief, musste bei der Verwendung von DMA deutlich mehr Energie zugeführt werden, und die Reaktion lief erst bei 116 °C ab

**Patentansprüche**

1. Computerimplementiertes Verfahren zur Berechnung von Übergangszustanden einer chemischen Reaktion umfassend die Schritte

   **A Erzeugen einer Startgeometrie**

   A1 Bereitstellung der dreidimensionalen Darstellung mindestens eines Molekuls im energetischen Grundzustand,
   A2 Auswahl von mindestens einer Bindung des mindestens einen Molekuls und Auswahl einer Länge der Bindung, wobei die ausgewählte Lange nicht der Lange der Bindung im energetischen Grundzustand des Molekuls entspricht, so dass eine Startgeometrie für die chemische Reaktion erhalten wird,
   A3 Dreidimensionale Darstellung der Startgeometrie in kartesischen und/oder internen Koordinaten,

   **B Ermittlung einer optimierten Startgeometrie**

   B1 Festlegung eines Funktionsraumes welcher die mindestens eine Bindung aus Schritt A2 und die durch diese Bindung verbundenen Atome umfasst,
   B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Lange der mindestens einen Bindung konstant gehalten wird, so dass eine optimierte Startgeometrie erhalten wird,
   B3 Ermittlung der Gradient-Norm B3 für die optimierte Startgeometrie, wobei die Gradient-Norm durch die erste Ableitung einer Funktion $E = f(x)$ mittels der quantenchemischen Methode erhalten wird, mit E=Gesamtenergie der optimierten Startgeometrie und x=Kernkoordinaten des Moleküls in der optimierten Startgeometrie,

      B4 1 wenn die Gradient-Norm B3 $\nabla$ $0 \leq \nabla < 0{,}07$ $E_h$ $a_0^{-1}$ betragt, dann Einordnung der optimierten Startgeometrie als eine Vorstufe für den Übergangszustand der chemischen Reaktion und Fortführung des Verfahrens mit Schritt D1, oder
      B4.2 wenn die Gradient-Norm B3 $\nabla > 0{,}07$ $E_h$ $a_0^{-1}$ betragt, dann Ermittlung der Vorstufe für den Übergangszustand der chemischen Reaktion ausgehend von der optimierten Startgeometrie durch ein Verfahren umfassend die folgenden Schritte.

   **C Ermittlung der Vorstufe für den Übergangszustand der chemischen Reaktion**

   C1 Variation der optimierten Startgeometrie unter Anwendung eines Monte-Carlo-Algorithmus wobei

      C11 wenigstens ein Atom aus dem in Schritt B1 ausgewahlten Funktionsraum zufällig ausgewählt wird,
      C1 2 ein Vektor für eine Auslenkung des in Schritt Cl.1 gewählten Atoms zufallig ausgewählt wird,
      C1 3 das in Schritt C1 1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

   C2 Geometrieoptimierung der Vorstufe für den Ubergangszustand mittels der quantenchemischen Methode und mit der Randbedingung, dass die mindestens eine Bindung aus Schritt A2 die Länge hat, die in der Schritt C1 3 ermittelt wurde,
   C3 Ermittlung der Gradient-Norm C3 für die Vorstufe für den Ubergangszustand aus Schritt C2, wobei die Gradient-Norm durch die erste Ableitung der Funktion $E = f(x)$ mittels der quantenchemischen Methode erhalten wird, mit E=Gesamtenergie der Vorstufe für den Übergangszustand und x=Kernkoordinaten des Molekuls in der Vorstufe für den Übergangszustand,
   C4 1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder
C4.2 wenn die Gradient-Norm C3 $\nabla$ > 0,07 $E_h\ a_0^{-1}$ betragt, dann Wiederholung der Schritte C1 bis C3 bis eine Gradient-Norm C3 $0 \le \nabla \le 0,07\ E_h\ a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder
(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den vorrangegangen Wiederholungen varnert wird, welche im Vergleich zu allen bisher erhaltenen Giadient-Normen C3 und B3 den niedrigsten Wert für die Gradient-Norm C3 oder B3 aufweist,

**D Ermittlung des Übergangszustandes**

D1 Relaxation der Vorstufe aus Schritt C4 1 oder der Vorstufe aus Schritt B4.1 mittels der quantenchemischen Methode und eines Pseudo-Newton-Raphson-Algorithmus, so dass der Übergangszustand erhalten wird,
D2 gegebenenfalls Ermittlung eines Gleichgewichtszustands durch Auslenkung des Übergangszustands, so dass ein ausgelenkter Übergangszustand erhalten wird und Relaxation des ausgelenkten Übergangszustands mittels der quantenchemischen Methode, so dass ein Gleichgewichtszustand erhalten wird

2. Verfahren nach Anspruch 1, **dadurch gekennzeichnet, dass** das mindestens eine Molekül aus Schritt A1 eine Große von hochstens 100 Atomen aufweist und/oder dass die Länge der in Schritt **A2** ausgewählten Bindung höchstens 230 pm beträgt

3. Verfahren nach Anspruch 1 oder 2, **dadurch gekennzeichnet, dass** Schritt **CI.2** die weiteren folgenden Schritte umfasst:

C1 2a Bestimmung eines weiteren Atoms aus dem in Schritt B1 ausgewahlten Funktionsraum, das nicht mit dem in Schritt CI.1 ausgewählten Atom übereinstimmt,
C1 2b Auswahl der Richtung des Vektors, wobei die Position des in Schritt C1 2 a ausgewahltenAtoms die Richtung des Vektors bestimmt,
C1 2 c zufällige Auswahl der Lange des Vektors, wobei der Wert für die Länge des Vektors positive oder negative Werte annehmen kann

4. Verfahren nach einem der vorstehenden Anspruche, **dadurch gekennzeichnet, dass** in Schritt
**C4.1** die Gradient-Norm C3 $\le$ 0,05 $E_h\ a_0^{-1}$, bevorzugt C3 $\le$ 0,04 $E_h\ a_0^{-1}$, bevorzugter
$\le$ 0,03 $E_h\ a_0^{-1}$, ist und Schritt **C4.2** so lange durchgeführt wird, bis eine Gradient-Norm
C3 $\le$ 0,05 $E_h\ a_0^{-1}$, bevorzugt C3 $\le$ 0,04 $E_h\ a_0^{-1}$, bevorzugter $\le$ 0,03 $E_h\ a_0^{-1}$, erhalten wird

5. Verfahren nach einem der vorstehenden Ansprüche, **dadurch gekennzeichnet, dass** Schritt **C4.2** höchstens 50 Mal, bevorzugt hochstens 30 Mal, wiederholt wild.

6. Verfahren nach einem der vorstehenden Ansprüche, **dadurch gekennzeichnet, dass** bei der Durchführung von Schritt **C4.2** bei der Wiederholung von Schritt **C1** ein anderes Atom ausgewahlt werden kann als bei der vorrangegangenen Durchführung des Verfahrens.

7. Verfahren nach einem der vorstehenden Anspruche, **dadurch gekennzeichnet, dass** die quantenchemische Methode aus Schritten **B2, B3, C2, C3, D1** und **D2** eine semiempirische Methode, Dichte-Funktional-theoretische Methode oder eine Annäherung der Schrödinger-Gleichung ist, insbesondere ist die quantenchemische Methode aus Schritten **B2, B3, C2, C3, D1** und **D2** eine Dichte-Funktional-theoretische Methode

8. Verfahren nach einem der vorstehenden Ansprüche, **dadurch gekennzeichnet, dass** die chemische Reaktion eine Synthese ist ausgewählt aus der Gruppe bestehend aus Polymersynthesen, insbesondere Polyurethansynthesen, Synthesen von Monomeren für Polymerisationsreaktionen, industriell benötigte Grundchemikalien, Additiven, Tensiden und pharmakologischen Wirkstoffen

9. Verfahren nach einem der vorstehenden Anspruche, **dadurch gekennzeichnet, dass** in Schritt **A1** mindestens zwei Moleküle I und II bereitgestellt werden und m Schritt **A2** alternativ oder zusatzlich zu der mindestens einen Bindung auch mindestens ein Abstand zwischen mindestens einem Atom aus Molekül I und mindestens einem Atom aus Molekül II sowie die Lange des mindestens einen Abstands ausgewahlt werden können, wobei die Länge des Abstands insbesondere hochstens 230 pm beträgt

10. Verfahren nach Anspruch 9, **dadurch gekennzeichnet, dass** die unter den Buchstaben **A, B,** und **C** des Verfahrens genannten Schritte wiederholt werden, wobei bei jeder Wiederholung im Vergleich zur vorrangegangenen Durchführungen des Verfahrens Molekul I verandert wird oder ein anderes Molekul als Molekül I bereitgestellt wird, und Molekül II nicht verandert wird und auch kein anderes Molekul als Molekül II bereitgestellt wird,
und umfassend einen zusätzlichen Schritt
**D0** Vergleich der bei den Wiederholungen erhaltenen Gradienten-Norm C3 für die verschiedenen Vorstufen für den Ubergangszustand und Auswahl der Vorstufe für den Übeigangszustand mit der niedrigsten Gradienten-Norm C3 und Durchführung von Schritt D1 und/oder D2 mit der ausgewählten Vorstufe für den Übergangszustand.

11. Verfahren nach Anspruch 9 oder 10, **dadurch gekennzeichnet, dass** Molekul I ein Katalysator für die chemische Reaktion ist, insbesondere für eine Polymersynthese, und Molekul II ein Edukt der chemischen Reaktion

12. Verfahren nach einem der Anspruche 9 bis 11, **dadurch gekennzeichnet, dass** die Länge des in Schritt **A2** ausgewahlten Abstands zwischen mindestens einem Atom aus Molekul I und/oder mindestens einem Atom aus Molekül II höchstens 230 pm beträgt

13. Verfahren nach einem der Ansprüche 9 bis 12, **dadurch gekennzeichnet, dass** Molekul I eine Größe von $\leq 100$ Atomen aufweist und Molekül II eine Große von $\leq 100$ Atomen aufweist, bevorzugt soll die Summe der Atome aus Molekül I und aus Molekul II $\leq 100$ Atome betragen.

14. System zur Datenverarbeitung umfassend Mittel zur Ausführung eines Verfahrens umfassend die Schritte.

### A Erzeugen einer Startgeometrie

A1 Bereitstellung der dreidimensionalen Darstellung mindestens eines Molekuls im energetischen Grundzustand,
A2 Auswahl von mindestens einer Bindung des mindestens einen Moleküls und Auswahl einer Lange der Bindung, wobei die ausgewählte Länge nicht der Länge der Bindung im energetischen Grundzustand des Molekuls entspricht, so dass eine Startgeometrie für die chemische Reaktion erhalten wird,
A3 Dreidimensionale Darstellung der Startgeometrie in kartesischen und/oder internen Koordinaten,

### B Ermittlung einer optimierten Startgeometrie

B1 Festlegung eines Funktionsraumes welcher die mindestens eine Bindung aus Schritt A2 und die durch diese Bindung verbundenen Atome umfasst,
B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Länge der mindestens einen Bindung konstant gehalten wird, so dass eine optimierte Startgeometrie erhalten wird,
B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,
B4.1 wenn die Gradient-Norm B3 $\nabla$ $0 \leq \nabla \leq 0{,}07$ $E_h\, a_0^{-1}$ betragt, dann Einordnung der optimierten Startgeometrie als eine Vorstufe für den Übergangszustand der chemischen Reaktion und Fortführung des Verfahrens mit Schritt D1, oder
B4 2 wenn die Gradient-Norm B3 $\nabla > 0{,}07$ $E_h\, a_0^{-1}$ beträgt, dann Ermittlung der Vorstufe für den Ubergangszustand der chemischen Reaktion ausgehend von der optimierten Startgeometrie durch ein Verfahren umfassend die folgenden Schritte:

### C Ermittlung der Vorstufe für den Übergangszustand der chemischen Reaktion

C1 Variation der optimierten Startgeometrie unter Anwendung eines Monte-Carlo-Algorithmus wobei

C1 1 wenigstens ein Atom aus dem in Schritt B1 ausgewählten Funktionsraum zufällig ausgewählt wird,

C1.2 ein Vektor für eine Auslenkung des in Schritt C1.1 gewählten Atoms zufällig ausgewählt wird,

C1 3 das in Schritt C1 1 ausgewählte Atom anhand des Vektors aus Schritt C1.2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Ubergangszustand mittels der quantenchemischen Methode und mit der Randbedingung, dass die mindestens eine Bindung aus Schritt A2 die Länge hat, die in der Schritt C1.3 ermittelt wurde,

C3 Ermittlung der Gradient-Norm C3 für die Vorstufe für den Ubergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Ubergangszustand und x=Kernkoordinaten des Molekuls in der Vorstufe für den Ubergangszustand,

C4 1 wenn die Gradient-Norm C3 $\nabla$ $0 \leq \nabla \leq 0{,}07$ $E_h$ $a_0^{-1}$ beträgt, dann Fortführung des Verfahrens mit Schritt D1, oder

C4.2 wenn die Gradient-Norm C3 $\nabla > 0{,}07$ $E_h$ $a_0^{-1}$ beträgt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Ubergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Ubergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Ubergangszustand erhalten wird,

D2 gegebenenfalls Ermittlung eines Gleichgewichtszustands durch Auslenkung des Ubergangszustands, so dass ein ausgelenkter Ubergangszustand erhalten wird und Relaxation des ausgelenkten Ubergangszustands mittels der quantenchemischen Methode, so dass ein Gleichgewichtszustand erhalten wird.

**15.** Computerprogramm, umfassend Befehle, die bei der Ausführung des Programms durch einen Computer diesen veranlassen, die folgenden Schritte eines Verfahrens durchzuführen.

**Optional A Erzeugen einer Startgeometrie**

A1 Bereitstellung der dreidimensionalen Darstellung mindestens eines Molekuls im energetischen Grundzustand,

A2 Auswahl von mindestens einer Bindung des mindestens einen Molekuls und Auswahl einer Lange der Bindung, wobei die ausgewählte Lange nicht der Länge der Bindung im energetischen Grundzustand des Molekuls entspricht, so dass eine Startgeometrie für die chemische Reaktion erhalten wird,

A3 Dreidimensionale Darstellung der Startgeometrie m kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewählten Funktionsraums mittels einer quantenchemischen Methode und mit der Randbedingung, dass die in Schritt A2 ausgewählte Lange der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ 0 $\leq$ $\nabla$ $\leq$ 0,07 $E_h$ $a_0^{-1}$ beträgt, dann Einordnung der optimierten Startgeometrie als eine Vorstufe für den Ubergangszustand der chemischen Reaktion und Fortführung des Verfahrens mit Schritt D1, oder

B4.2 wenn die Gradient-Norm B3 $\nabla$ > 0,07 $E_h$ $a_0^{-1}$ betragt, dann Ermittlung der Vorstufe für den Ubergangszustand der chemischen Reaktion ausgehend von der optimierten Startgeometrie durch ein Verfahren umfassend die folgenden Schritte:

**C Ermittlung der Vorstufe für den Übergangszustand der chemischen Reaktion**

C1 Variation der optimierten Startgeometrie unter Anwendung eines Monte-Carlo-Algorithmus wobei

C1 1 wenigstens ein Atom aus dem in Schritt B1 ausgewählten Funktionsraum zufällig ausgewählt wird,

C1.2 ein Vektor für eine Auslenkung des in Schritt C1.1 gewählten Atoms zufällig ausgewählt wird,

C1.3 das in Schritt C1 1 ausgewahlte Atom anhand des Vektors aus Schritt C1 2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schritt A2 die Länge hat, die in der Schritt C1 3 ermittelt wurde,

C3 Ermittlung der Gradient-Norm C3 für die Vorstufe für den Ubergangszustand aus Schritt C2, wobei die Gradient-Norm durch die eiste Ableitung der Funktion E = f(x) mittels der quantenchemischen Methode erhalten wird, mit E=Gesamtenergie der Vorstufe für den Übergangszustand und x=Kernkoordinaten des Molekuls in der Vorstufe für den Ubergangszustand,

C4 1 wenn die Gradient-Norm C3 $\nabla$ 0 $\leq$ $\nabla$ $\leq$ 0,07 $E_h$ $a_0^{-1}$ beträgt, dann Fortführung des Verfahrens mit Schritt D1, oder

C4 2 wenn die Gradient-Norm C3 $\nabla$ > 0,07 $E_h$ $a_0^{-1}$ beträgt, dann Wiederholung der Schritte C1 bis C3 bis eine Gradient-Norm C3 0 $\leq$ $\nabla$ $\leq$ 0,07 $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Ubergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Ubergangszustand aus den vorrangegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4 1 oder der Vorstufe aus Schritt B4 1 mittels der quantenchemischen Methode und eines Pseudo-Newton-Raphson-Algonthmus, so dass der Übergangszustand erhalten wird,

D2 gegebenenfalls Ermittlung eines Gleichgewichtszustands durch Auslenkung des Ubergangszustands, so dass ein ausgelenkter Ubergangszustand erhalten wird und Relaxation des ausgelenkten Übergangszustands mittels der quantenchemischen Methode, so dass ein Gleichgewichtszustand erhalten wird.

**16.** Computerlesbares Speichermedium, umfassend Befehle, die bei der Ausführung durch einen Computer diesen veranlassen, die folgenden Schritte eines Verfahrens durchzuführen:

**Optional A Erzeugen einer Startgeometrie**

A1 Bereitstellung der dreidimensionalen Darstellung mindestens eines Moleküls im energetischen Grundzustand,

A2 Auswahl von mindestens einen Bindung des mindestens einen Moleküls und Auswahl einer Länge der Bindung, wobei die ausgewählte Länge nicht der Länge der Bindung im energetischen Grundzustand des Molekuls entspricht, so dass eine Startgeometrie für die chemische Reaktion erhalten wird,

A3 Dreidimensionale Darstellung der Startgeometrie in kartesischen und/oder internen Koordinaten,

**B Ermittlung einer optimierten Startgeometrie**

B1 Festlegung eines Funktionsraumes welcher die mindestens eine Bindung aus Schritt A2 und die durch diese Bindung verbundenen Atome umfasst,

B2 Geometrieoptimierung der Startgeometrie unter Zugrundelegung des in Schritt B1 ausgewahlten Funktionsraums mittels einer quantenchemischen Methode und mit der Randbedingung, dass die in Schritt A2 ausgewahlte Länge der mindestens einen Bindung konstant gehalten wird, so dass eine optimierte Startgeometrie erhalten wird,

B3 Ermittlung der Gradient-Norm B3 für die optimierte Startgeometrie, wobei die Gradient-Norm durch die erste Ableitung einer Funktion E = f(x) mittels der quantenchemischen Methode erhalten wird, mit E=Gesamtenergie der optimierten Startgeometrie und x=Kernkoordinaten des Moleküls in der optimierten Startgeometrie,

B4.1 wenn die Gradient-Norm B3 $\nabla$ $0 \leq \nabla \leq 0{,}07$ $E_h$ $a_0^{-1}$ betragt, dann Emordnung der optimierten Startgeometrie als eine Vorstufe für den Übergangszustand der chemischen Reaktion und Fortführung des Verfahrens mit Schritt D1, oder

B4 2 wenn die Gradient-Norm B3 $\nabla > 0{,}07$ $E_h$ $a_0^{-1}$ betragt, dann Ermittlung der Vorstufe für den Übergangszustand der chemischen Reaktion ausgehend von der optimierten Startgeometrie durch ein Verfahren umfassend die folgenden Schritte

**C Ermittlung der Vorstufe für den Übergangszustand der chemischen Reaktion**

C1 Variation der optimierten Startgeometrie unter Anwendung eines Monte-Carlo-Algorithmus wobei

C1.1 wenigstens ein Atom aus dem in Schritt B1 ausgewahlten Funktionsraum zufällig ausgewählt wird,

C1 2 ein Vektor für eine Auslenkung des in Schritt C1.1 gewählten Atoms zufällig ausgewählt wird,

C1 3 das in Schritt C1 1 ausgewählte Atom anhand des Vektors aus Schritt C1 2 aus seiner Position in der optimierten Startgeometrie ausgelenkt wird, so dass eine Vorstufe für den Übergangszustand der chemischen Reaktion erhalten wird,

C2 Geometrieoptimierung der Vorstufe für den Übergangszustand mittels der quantenchemischen Methode und mit der Randbedingung, dass die mindestens eine Bindung aus Schi itt A2 die Länge hat, die in der Schritt C1 3 ermittelt wurde,

C3 Ermittlung der Gradient-Norm C3 für die Vorstufe für den Ubergangszustand aus Schritt C2, wobei die Gradient-Norm durch die erste Ableitung der Funktion E = f(x) mittels der quantenchemischen Methode erhalten wird, mit E=Gesamtenergie der Vorstufe für den Übergangszustand und x=Kernkoordinaten des Moleküls in der Vorstufe für den Ubergangszustand,

C4 1 wenn die Gradient-Norm C3 $\nabla$ $0 \leq \nabla \leq 0{,}07$ $E_h$ $a_0^{-1}$ betragt, dann Fortführung des Verfahrens mit Schritt D1, oder

C4 2 wenn die Gradient-Norm C3 $\nabla > 0{,}07$ $E_h$ $a_0^{-1}$ beträgt, dann Wiederholung der Schritte C1 bis C3 bis eine Gradient-Norm C3 $0 \leq \nabla \leq 0{,}07$ $E_h$ $a_0^{-1}$ erhalten wird, wobei

(a) für den Fall, dass die Schritte C1 bis C3 ein Mal durchgeführt wurden, in Schritt C1 die Geometrie der Vorstufe für den Übergangszustand variiert wird, wenn der Wert ihrer Gradient-Norm C3 niedriger ist als der Wert der Gradient-Norm B3 der optimierten Startgeometrie, oder

(b) für den Fall dass die Schritte C1 bis C3 mehr als ein Mal durchgeführt wurden, in Schritt C1 die Geometrie der optimierten Startgeometrie oder derjenigen Vorstufe für den Übergangszustand aus den von angegangen Wiederholungen variiert wird, welche im Vergleich zu allen bisher erhaltenen Gradient-Normen C3 und B3 den niedrigsten Wert für die Gradient-Norm C3 oder B3 aufweist,

**D Ermittlung des Übergangszustandes**

D1 Relaxation der Vorstufe aus Schritt C4.1 oder der Vorstufe aus Schritt B4.1 mittels der quantenchemischen Methode und eines Pseudo-Newton-Raphson-Algorithmus, so dass der Übergangszustand erhalten wird,

D2 gegebenenfalls Ermittlung eines Gleichgewichtszustands durch Auslenkung des Ubergangszustands, so dass ein ausgelenkter Übergangszustand erhalten wird und Relaxation des ausgelenkten Übergangszustands mittels der quantenchemischen Methode, so dass ein Gleichgewichtszustand erhalten wird

17. Verwendung eines Computerprogramms nach Anspruch 15 oder eines computerlesbaren Speichermediums nach Anspruch 16 zur Bewertung von Übergangszuständen einer chemischen Reaktion, insbesondere einer Polymer-synthese.

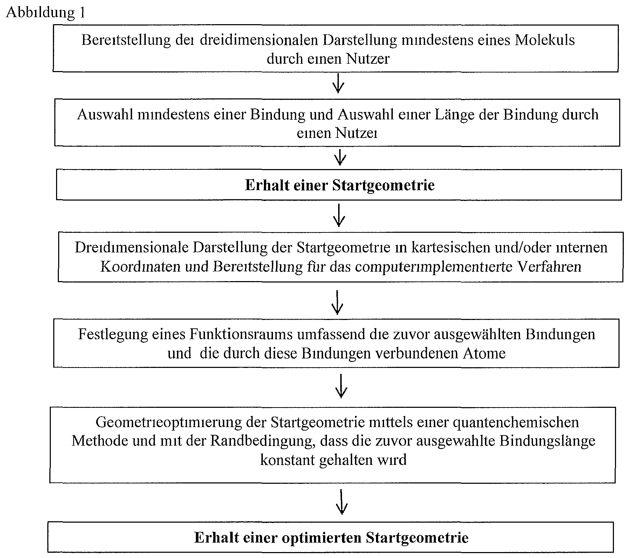

Abbildung 1

| Bereitstellung dei dreidimensionalen Darstellung mindestens eines Molekuls durch einen Nutzer |
|---|

↓

| Auswahl mindestens einer Bindung und Auswahl einer Länge der Bindung durch einen Nutzei |
|---|

↓

| **Erhalt einer Startgeometrie** |
|---|

↓

| Dreidimensionale Darstellung der Startgeometrie in kartesischen und/oder internen Koordinaten und Bereitstellung für das computerimplementierte Verfahren |
|---|

↓

| Festlegung eines Funktionsraums umfassend die zuvor ausgewählten Bindungen und die durch diese Bindungen verbundenen Atome |
|---|

↓

| Geometrieoptimierung der Startgeometrie mittels einer quantenchemischen Methode und mit der Randbedingung, dass die zuvor ausgewahlte Bindungslänge konstant gehalten wird |
|---|

↓

| **Erhalt einer optimierten Startgeometrie** |
|---|

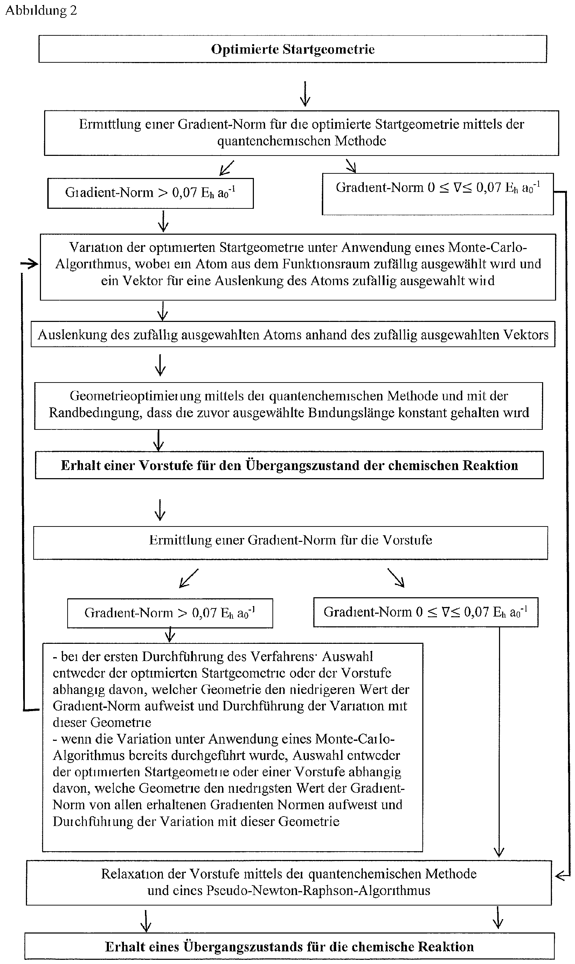

Abbildung 2

| Optimierte Startgeometrie |

Ermittlung einer Gradient-Norm für die optimierte Startgeometrie mittels der quantenchemischen Methode

Gradient-Norm $> 0{,}07$ $E_h$ $a_0^{-1}$

Gradient-Norm $0 \leq \nabla \leq 0{,}07$ $E_h$ $a_0^{-1}$

Variation der optimierten Startgeometrie unter Anwendung eines Monte-Carlo-Algorithmus, wobei ein Atom aus dem Funktionsraum zufällig ausgewählt wird und ein Vektor für eine Auslenkung des Atoms zufällig ausgewählt wird

Auslenkung des zufällig ausgewählten Atoms anhand des zufällig ausgewählten Vektors

Geometrieoptimierung mittels der quantenchemischen Methode und mit der Randbedingung, dass die zuvor ausgewählte Bindungslänge konstant gehalten wird

**Erhalt einer Vorstufe für den Übergangszustand der chemischen Reaktion**

Ermittlung einer Gradient-Norm für die Vorstufe

Gradient-Norm $> 0{,}07$ $E_h$ $a_0^{-1}$

Gradient-Norm $0 \leq \nabla \leq 0{,}07$ $E_h$ $a_0^{-1}$

- bei der ersten Durchführung des Verfahrens: Auswahl entweder der optimierten Startgeometrie oder der Vorstufe abhängig davon, welcher Geometrie den niedrigeren Wert der Gradient-Norm aufweist und Durchführung der Variation mit dieser Geometrie
- wenn die Variation unter Anwendung eines Monte-Carlo-Algorithmus bereits durchgeführt wurde, Auswahl entweder der optimierten Startgeometrie oder einer Vorstufe abhängig davon, welche Geometrie den niedrigsten Wert der Gradient-Norm von allen erhaltenen Gradienten Normen aufweist und Durchführung der Variation mit dieser Geometrie

Relaxation der Vorstufe mittels der quantenchemischen Methode und eines Pseudo-Newton-Raphson-Algorithmus

**Erhalt eines Übergangszustands für die chemische Reaktion**

Europäisches
Patentamt

European
Patent Office

Office européen
des brevets

# EUROPÄISCHER RECHERCHENBERICHT

Nummer der Anmeldung

EP 18 20 1340

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | LIN X-X. ET AL: "A flexible transition state searching method for atmospheric reaction systems", CHEMICAL PHYSICS, NORTH-HOLLAND, NL, Bd. 450, 11. Februar 2015 (2015-02-11), Seiten 21-31, XP029204426, ISSN: 0301-0104, DOI: 10.1016/J.CHEMPHYS.2015.02.002 * das ganze Dokument * | 1-17 | INV. G16C20/10 |
| A | SWIHART M. ET AL: "Assembling gas-phase reaction mechanisms for high temperature inorganic systems based on quantum chemistry calculations and reaction rate theories", JOURNAL OF PHYSICS AND CHEMISTRY OF SOLIDS, PERGAMON PRESS, LONDON, GB, Bd. 66, Nr. 2-4, 1. Februar 2005 (2005-02-01), Seiten 364-371, XP027709615, ISSN: 0022-3697 [gefunden am 2005-02-01] * Seite 365, linke Spalte, Zeile 36 - Seite 366, linke Spalte, Zeile 8 * | 1-17 | |
| A | HAFNER J.: "Adsorption and reaction of organic molecules on solid surfaces - ab-initio density functional investigations", MONATSHEFTE FÜR CHEMIE - CHEMICAL MONTHLY ; AN INTERNATIONAL JOURNAL OF CHEMISTRY, SPRINGER-VERLAG, AU, Bd. 139, Nr. 4, 14. März 2008 (2008-03-14) , Seiten 373-387, XP019592602, ISSN: 1434-4475 * Seite 375, rechte Spalte, Zeilen 10-19 * * Abbildungen 1,2,7,8 * | 1-17 | RECHERCHIERTE SACHGEBIETE (IPC) G16C |

-/--

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

Europäisches Patentamt

European Patent Office

Office européen des brevets

**EUROPÄISCHER RECHERCHENBERICHT**

Nummer der Anmeldung

EP 18 20 1340

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 2 352 107 A1 (UNIV YAMAGUCHI [JP]) 3. August 2011 (2011-08-03) * das ganze Dokument * | 1-17 | |
| A | JP 2008 250392 A (UNIV YAMAGUCHI) 16. Oktober 2008 (2008-10-16) * das ganze Dokument * | 1-17 | |
| A | CN 104 765 918 A (UNIV EAST CHINA SCIENCE & TECH) 8. Juli 2015 (2015-07-08) * das ganze Dokument * | 1-17 | |
| A,D | MARTÍNEZ-NÚÑEZ E.: "An automated transition state search using classical trajectories initialized at multiple minima", PHYSICAL CHEMISTRY CHEMICAL PHYSICS, Bd. 17, Nr. 22, 6. Mai 2015 (2015-05-06), Seiten 14912-14921, XP055601422, ISSN: 1463-9076, DOI: 10.1039/C5CP02175H * das ganze Dokument * | 1-17 | |
| A,D | RODRÍGUEZ A. ET AL: "tsscds2018: A code for automated discovery of chemical reaction mechanisms and solving the kinetics", JOURNAL OF COMPUTATIONAL CHEMISTRY., Bd. 39, Nr. 23, 5. September 2018 (2018-09-05), Seiten 1922-1930, XP055601919, GB ISSN: 0192-8651, DOI: 10.1002/jcc.25370 * das ganze Dokument * | 1-17 | RECHERCHIERTE SACHGEBIETE (IPC) |

-/--

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**Europäisches Patentamt**
**European Patent Office**
**Office européen des brevets**

# EUROPÄISCHER RECHERCHENBERICHT

Nummer der Anmeldung

EP 18 20 1340

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | JACOBSON L. D.ET AL: "Automated Transition State Search and Its Application to Diverse Types of Organic Reactions", JOURNAL OF CHEMICAL THEORY AND COMPUTATION: JCTC, Bd. 13, Nr. 11, 17. Oktober 2017 (2017-10-17), Seiten 5780-5797, XP055601412, US ISSN: 1549-9618, DOI: 10.1021/acs.jctc.7b00764 * das ganze Dokument * ----- | 1-17 | |
| A | SALCICCIOLI M. ET AL: "A review of multiscale modeling of metal-catalyzed reactions: Mechanism development for complexity and emergent behavior", CHEMICAL ENGINEERING SCIENCE, OXFORD, GB, Bd. 66, Nr. 19, 30. Mai 2011 (2011-05-30), Seiten 4319-4355, XP028264661, ISSN: 0009-2509, DOI: 10.1016/J.CES.2011.05.050 [gefunden am 2011-06-13] * Zusammenfassung * * Abbildungen 11,26,27 * ----- | 1-17 | RECHERCHIERTE SACHGEBIETE (IPC) |
| | -/-- | | |

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

                                                           
& : Mitglied der gleichen Patentfamilie, übereinstimmendes Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 18 20 1340

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | KEIL F. J.: "Complexities in modeling of heterogeneous catalytic reactions", COMPUTERS & MATHEMATICS WITH APPLICATIONS, ELSEVIER, AMSTERDAM, NL, Bd. 65, Nr. 10, 3. Januar 2013 (2013-01-03), Seiten 1674-1697, XP028556589, ISSN: 0898-1221, DOI: 10.1016/J.CAMWA.2012.11.023 * Zusammenfassung * * Seite 1688, Zeilen 13-16 * * Seite 1692, Zeilen 15-38 * * Abbildung 19 * ----- | 1-17 | |
| A | DIEDRICH M. K. ET AL: "Experimental Determination of the Activation Parameters and Stereoselectivities of the Intramolecular Diels-Alder Reactions of 1,3,8-Nonatriene, 1,3,9-Decatriene, and 1,3,10-Undecatriene and Transition State Modeling with the Monte Carlo-Jumping Between Wells/Molecular Dynamics Method", JOURNAL OF THE AMERICAN CHEMICAL SOCIETY, Bd. 119, Nr. 43, 1. Oktober 1997 (1997-10-01), Seiten 10255-10259, XP055593729, ISSN: 0002-7863, DOI: 10.1021/ja9643331 * Zusammenfassung * * Seite 10256, rechte Spalte, Zeile 48 - Seite 10259, linke Spalte, Zeile 7 * ----- | 1-17 | RECHERCHIERTE SACHGEBIETE (IPC) |
| A | US 2003/236655 A1 (GOVIND NIRANJAN [US] ET AL) 25. Dezember 2003 (2003-12-25) * das ganze Dokument * ----- | 1-17 | |
| A | JP H11 25063 A (HITACHI LTD) 29. Januar 1999 (1999-01-29) * das ganze Dokument * ----- | 1-17 | |

-/--

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes Dokument

EPO FORM 1503 03.82 (P04C03)

Seite 4 von 6

Europäisches
Patentamt

European
Patent Office

Office européen
des brevets

# EUROPÄISCHER RECHERCHENBERICHT

Nummer der Anmeldung

EP 18 20 1340

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2018/102565 A1 (SCHROEDINGER INC [US]) 7. Juni 2018 (2018-06-07) * das ganze Dokument * ----- | 1-17 | |
| A | MERCERO J. M. ET AL: "Theoretical methods that help understanding the structure and reactivity of gas phase ions", INTERNATIONAL JOURNAL OF MASS SPECTROMETRY, ELSEVIER SCIENCE PUBLISHERS, AMSTERDAM, NL, Bd. 240, Nr. 1, 1. Januar 2005 (2005-01-01), Seiten 37-99, XP027705154, ISSN: 1387-3806 [gefunden am 2005-01-01] * Seite 71, rechte Spalte, Zeilen 10-35 * ----- | 1-17 | |
| A | KRATZER P.: "Monte Carlo and kinetic Monte Carlo methods", ARXIV.ORG, CORNELL UNIVERSITY LIBRARY, 201 OLIN LIBRARY CORNELL UNIVERSITY ITHACA, NY 14853, 16. April 2009 (2009-04-16), XP080320087, * das ganze Dokument * ----- | 1-17 | RECHERCHIERTE SACHGEBIETE (IPC) |
| A | GREBNER C. ET AL: "PathOpt-A global transition state search approach: Outline of algorithm", JOURNAL OF COMPUTATIONAL CHEMISTRY., Bd. 34, Nr. 21, 4. Mai 2013 (2013-05-04), Seiten 1810-1818, XP055601427, GB ISSN: 0192-8651, DOI: 10.1002/jcc.23307 * das ganze Dokument * ----- | 1-17 | |

-/--

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
............................................................................
& : Mitglied der gleichen Patentfamilie, übereinstimmendes Dokument

EPO FORM 1503 03.82 (P04C03)

Europäisches
Patentamt

European
Patent Office

Office européen
des brevets

# EUROPÄISCHER RECHERCHENBERICHT

Nummer der Anmeldung

EP 18 20 1340

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | LIN Y. ET AL: "Reliable Modeling and Optimization for Chemical Engineering Applications: Interval Analysis Approach", RELIABLE COMPUTING ; AN INTERNATIONAL JOURNAL DEVOTED TO RELIABLE MATHEMATICAL COMPUTATIONS BASED ON FINITE REPRESENTATIONS AND GUARANTEED ACCURACY, KLUWER ACADEMIC PUBLISHERS, DO, Bd. 12, Nr. 6, 25. Oktober 2006 (2006-10-25), Seiten 427-450, XP019453859, ISSN: 1573-1340, DOI: 10.1007/S11155-006-9013-6 * Seite 442, Absatz 5. - Seite 447, Absatz 6. * ----- | 1-17 | |

RECHERCHIERTE
SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 8. Juli 2019 | Godzina, Przemyslaw |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

 

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 18 20 1340

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-07-2019

| Im Recherchenbericht angeführtes Patentdokument | Datum der Veröffentlichung | Mitglied(er) der Patentfamilie | Datum der Veröffentlichung |
|---|---|---|---|
| EP 2352107 A1 | 03-08-2011 | EP 2352107 A1<br>JP 5164111 B2<br>JP 2010097371 A<br>US 2011191390 A1<br>WO 2010044191 A1 | 03-08-2011<br>13-03-2013<br>30-04-2010<br>04-08-2011<br>22-04-2010 |
| JP 2008250392 A | 16-10-2008 | JP 4324680 B2<br>JP 2008250392 A | 02-09-2009<br>16-10-2008 |
| CN 104765918 A | 08-07-2015 | KEINE | |
| US 2003236655 A1 | 25-12-2003 | CA 2431474 A1<br>US 2003236655 A1 | 24-12-2003<br>25-12-2003 |
| JP H1125063 A | 29-01-1999 | KEINE | |
| WO 2018102565 A1 | 07-06-2018 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **LIN et al.** A flexible transition state searching method for atmospheric reaction systems. *Chemical Physics,* 2015, vol. 450-451, S 21-31
- **E. MARTÍNEZ-NÚÑEZ et al.** An automated transition state search using classical trajectories initialized at multiple minima. *Phys Chem Chem Phys,* 14. Juni 2015, vol. 17 (22), 14912-21
- tsscds2018 A code for automated discovery of chemical reaction mechanisms and solving the kinetics. *J. Comp.Chem.,* 24. September 2018, vol. 39 (23), 1922-1930

- **JACOBSON et al.** Automated Transition State Search and Its Application to Diverse Types of Organic Reactions. *J. Chem Theory Comput.,* vol. 13 (11), 5780-5797
- **HU et al.** A gradient-directed Monte Carlo method for global optimization in a discrete space. Application to protein sequence design and folding. *J Chem Phys,* 21. Oktober 2009, vol. 131 (15), 154117